# EUROPÄISCHE PATENTSCHRIFT

(11) **EP 0 751 995 B1**
(45) Veröffentlichungstag und Bekanntmachung des Hinweises auf die Patenterteilung: **18.02.2004**
(21) Anmeldenummer: 95913119.4
(22) Anmeldetag: 15.03.1995
(51) Int. Cl.: C12N 15/52, C12N 15/53, C12N 15/54, C12N 15/55, C12N 15/81, C12N 1/19, C12P 25/00

(54) **RIBOFLAVIN-BIOSYNTHESIS IN FUNGI**
RIBOFLAVIN SYNTHESIS IN FUNGI
BIOSYNTHESE DE LA RIBOFLAVINE DANS DES CHAMPIGNONS

(30) Priorität: 25.03.1994 DE 4410382; 15.06.1994 DE 4420785
(43) Veröffentlichungstag der Anmeldung: 08.01.1997
(73) Patentinhaber: BASF AKTIENGESELLSCHAFT, 67056 Ludwigshafen (DE)
(72) Erfinder: REVUELTA DOVAL, Jose, Luis, E-37001 Salamanca (ES); BUITRAGO SERNA, Maria, Jose, E-37004 Salamanca (ES); SANTOS GARCIA, Maria, Angeles, E-37009 Santa Marta (ES)
(86) Internationale Anmeldenummer: PCT/EP1995/000958
(87) Internationale Veröffentlichungsnummer: WO 1995/026406

(56) Entgegenhaltungen:
- EP-A- 0 405 370
- EP-A- 0 569 806
- WO-A-94/11515
- YEAST, Bd. 9, Nr. 10, Oktober 1993 JOHN WILEY & SONS LTD., CHICHESTER, UK, Seiten 1099-1102, M.-J. BUITRAGO ET AL. 'Mapping of the RIB1 and RIB7 genes involved in the biosynthesis of riboflavin in Saccharomyces cerevisiae'
- YEAST (1993), 9(2), 189-99 CODEN: YESTE3;ISSN: 0749-503X, 1993 DOIGNON, FRANCOIS ET AL 'The complete sequence of a 19,482 bp segment located on the right arm of chromosome II from Saccharomyces cerevisiae'

### SEQUENZPROTOKOLL

### (1) ALGEMEINE INFORMATION:

(i) ANMELDER:
   (A) NAME: BASF Aktiengesellschaft
   (B) STRASSE: Carl-Bosch-Strasse 38
   (C) ORT: Ludwigshafen
   (E) LAND: Bundesrepublik Deutschland
   (F) POSTLEITZAHL: D-67056
   (G) TELEPHON: 0621/6048526
   (H) TELEFAX: 0621/6043123
   (I) TELEX: 1762175170
(ii) ANMELDETITEL: Riboflavin-Biosynthese in Pilzen
(iii) ANZAHL DER SEQUENZEN: 12
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.25 (EPA)

### (2) INFORMATION ZU SEQ ID NO: 1:

(i) SEQUENZ CHARAKTERISTIKA:
   (A) LÄNGE: 1329 Basenpaare
   (B) ART: Nukleinsäure
   (C) STRANGFORM: Doppel
   (D) TOPOLOGIE: linear
(ii) ART DES MOLEKÜLS: cDNS zu mRNS
(iii) HYPOTHETISCH: NEIN
(iii) ANTISENSE: NEIN
(vi) URSPRÜNLICHE HERKUNFT:
   (A) ORGANISMUS: Ashbya gossypii
(ix) MERKMALE:
   (A) NAME/SCHLÜSSEL: 5'UTR
   (B) LAGE: 1..242
(ix) MERKMALE:
   (A) NAME/SCHLÜSSEL: CDS
   (B) LAGE: 243..1148
(ix) MERKMALE:
   (A) NAME/SCHLÜSSEL: 3'UTR
   (B) LAGE: 1149..1329
(xi) SEQUENZBESCHREIBUNG: SEQ ID NO: 1:
   TTTCTGTCCG CATACTTCAT ATGCTCATCG CACATTGATA ATGTACATTC GAAAAATTTC      60
   AAGATTAGCC TCCGTGAACA GCGATTTACC TTAGGCAAAA GTAACAAAAG GCTTTTCCGT     120
   AGGTGCTTTG TCATTCAACA ATCCACGTCG GAATTGGCGA CTATATAGTG TAGGGCCCAT     180
   AAAGCAGTAG TCGGTGTTGA TAGCTGTGTC AGACCAACTC TTTGTTAATT ACTGAAGCTG     240
   AT ATG ACT GAA TAC ACA GTG CCA GAA GTG AGG TGT GTC GCA CGC GCG        287
      Met Thr Glu Tyr Thr Val Pro Glu Val Arg Cys Val Ala Arg Ala
       1               5                  10                  15
   CGC ATA CCG ACG GTA CAG GGC ACC GAT GTC TTC CTC CAT CTA TAC CAC        335
   Arg Ile Pro Thr Val Gln Gly Thr Asp Val Phe Leu His Leu Tyr His
                       20                  25                  30
   AAC TCG ATC GAC AGC AAG GAA CAC CTA GCG ATT GTC TTC GGC GAG AAC        383
   Asn Ser Ile Asp Ser Lys Glu His Leu Ala Ile Val Phe Gly Glu Asn
                   35                  40                  45
   ATA CGC TCG CGG AGT CTG TTC CGG TAC CGG AAA GAC GAC ACG CAG CAG        431
   Ile Arg Ser Arg Ser Leu Phe Arg Tyr Arg Lys Asp Asp Thr Gln Gln
               50                  55                  60
   GCG CGG ATG GTG CGG GGC GCC TAC GTG GGC CAG CTG TAC CCC GGG CGG        479
   Ala Arg Met Val Arg Gly Ala Tyr Val Gly Gln Leu Tyr Pro Gly Arg
       65                  70                  75
   ACC GAG GCA GAC GCG GAT CGG CGT CAG GGC CTG GAG CTG CGG TTT GAT        527
   Thr Glu Ala Asp Ala Asp Arg Arg Gln Gly Leu Glu Leu Arg Phe Asp
   80                  85                  90                  95
   GAG ACA GGG CAG CTG GTG GTG GAG CGG GCG ACG ACG TGG ACC AGG GAG        575
   Glu Thr Gly Gln Leu Val Val Glu Arg Ala Thr Thr Trp Thr Arg Glu
                   100                 105                 110
   CCG ACA CTG GTG CGG CTG CAC TCG GAG TGT TAC ACG GGC GAG ACG GCG        623
   Pro Thr Leu Val Arg Leu His Ser Glu Cys Tyr Thr Gly Glu Thr Ala
               115                 120                 125
   TGG AGC GCG CGG TGC GAC TGC GGG GAG CAG TTC GAC CAG GCG GGT AAG        671
   Trp Ser Ala Arg Cys Asp Cys Gly Glu Gln Phe Asp Gln Ala Gly Lys
           130                 135                 140
   CTG ATG GCT GCG GCG ACA GAG GGC GAG GTG GTT GGC GGT GCG GGG CAC        719
   Leu Met Ala Ala Ala Thr Glu Gly Glu Val Val Gly Gly Ala Gly His
       145                 150                 155
   GGC GTG ATC GTG TAC CTG CGG CAG GAG GGC CGC GGC ATC GGG CTA GGC        767
   Gly Val Ile Val Tyr Leu Arg Gln Glu Gly Arg Gly Ile Gly Leu Gly
   160                 165                 170                 175
   GAG AAG CTG AAG GCG TAC AAC CTG CAG GAC CTG GGC GCG GAC ACG GTG        815
   Glu Lys Leu Lys Ala Tyr Asn Leu Gln Asp Leu Gly Ala Asp Thr Val
                   180                 185                 190
   CAG GCG AAC GAG CTG CTC AAC CAC CCT GCG GAC GCG CGC GAC TTC TCG        863
   Gln Ala Asn Glu Leu Leu Asn His Pro Ala Asp Ala Arg Asp Phe Ser
               195                 200                 205
   TTG GGG CGC GCA ATC CTA CTG GAC CTC GGT ATC GAG GAC ATC CGG TTG        911
   Leu Gly Arg Ala Ile Leu Leu Asp Leu Gly Ile Glu Asp Ile Arg Leu
           210                 215                 220
   CTC ACG AAT AAC CCC GAC AAG GTG CAG CAG GTG CAC TGT CCG CCG GCG        959
   Leu Thr Asn Asn Pro Asp Lys Val Gln Gln Val His Cys Pro Pro Ala
       225                 230                 235
   CTA CGC TGC ATC GAG CGG GTG CCC ATG GTG CCG CTT TCA TGG ACT CAG       1007
   Leu Arg Cys Ile Glu Arg Val Pro Met Val Pro Leu Ser Trp Thr Gln
   240                 245                 250                 255
   CCC ACA CAG GGC GTG CGC TCG CGC GAG CTG GAC GGC TAC CTG CGC GCC      1055
   Pro Thr Gln Gly Val Arg Ser Arg Glu Leu Asp Gly Tyr Leu Arg Ala
                   260                 265                 270
   AAG GTC GAG CGC ATG GGG CAC ATG CTG CAG CGG CCG CTG GTG CTG CAC      1103
   Lys Val Glu Arg Met Gly His Met Leu Gln Arg Pro Leu Val Leu His
               275                 280                 285
   ACG TCT GCG GCG GCC GAG CTC CCC CGC GCC AAC ACA CAC ATA TAATCTTTGC   1155
   Thr Ser Ala Ala Ala Glu Leu Pro Arg Ala Asn Thr His Ile
           290                 295                 300
   TATATTAAAA CTCTATAAAC GTATGCCACA CGGCGCCCGC GGGCTGCCAC ACGCTGCTCA    1215
   CGGGCTGCCG AACAGTTCTA ACAAGTAATC GCGCGCCTCG CCAGTGATCG TGGCGAGCAC    1275
   CTTGTCGTCC ATCATCACAT ATCCTCGGCT ACAGTCGTCG TTGAAGAGCG TGCA          1329

### (2) INFORMATION ZU SEQ ID NO: 2:

(i) SEQUENZ CHARAKTERISTIKA:
   (A) LÄNGE: 301 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 2:
   Met Thr Glu Tyr Thr Val Pro Glu Val Arg Cys Val Ala Arg Ala Arg
    1               5                  10                  15
   Ile Pro Thr Val Gln Gly Thr Asp Val Phe Leu His Leu Tyr His Asn
               20                  25                  30
   Ser Ile Asp Ser Lys Glu His Leu Ala Ile Val Phe Gly Glu Asn Ile
           35                  40                  45
   Arg Ser Arg Ser Leu Phe Arg Tyr Arg Lys Asp Asp Thr Gln Gln Ala
       50                  55                  60
   Arg Met Val Arg Gly Ala Tyr Val Gly Gln Leu Tyr Pro Gly Arg Thr
   65                  70                  75                  80
   Glu Ala Asp Ala Asp Arg Arg Gln Gly Leu Glu Leu Arg Phe Asp Glu
                   85                  90                  95
   Thr Gly Gln Leu Val Val Glu Arg Ala Thr Thr Trp Thr Arg Glu Pro
               100                 105                 110
   Thr Leu Val Arg Leu His Ser Glu Cys Tyr Thr Gly Glu Thr Ala Trp
           115                 120                 125
   Ser Ala Arg Cys Asp Cys Gly Glu Gln Phe Asp Gln Ala Gly Lys Leu
       130                 135                 140
   Met Ala Ala Ala Thr Glu Gly Glu Val Val Gly Gly Ala Gly His Gly
   145                 150                 155                 160
   Val Ile Val Tyr Leu Arg Gln Glu Gly Arg Gly Ile Gly Leu Gly Glu
                   165                 170                 175
   Lys Leu Lys Ala Tyr Asn Leu Gln Asp Leu Gly Ala Asp Thr Val Gln
               180                 185                 190
   Ala Asn Glu Leu Leu Asn His Pro Ala Asp Ala Arg Asp Phe Ser Leu
           195                 200                 205
   Gly Arg Ala Ile Leu Leu Asp Leu Gly Ile Glu Asp Ile Arg Leu Leu
       210                 215                 220
   Thr Asn Asn Pro Asp Lys Val Gln Gln Val His Cys Pro Pro Ala Leu
   225                 230                 235                 240
   Arg Cys Ile Glu Arg Val Pro Met Val Pro Leu Ser Trp Thr Gln Pro
                   245                 250                 255
   Thr Gln Gly Val Arg Ser Arg Glu Leu Asp Gly Tyr Leu Arg Ala Lys
               260                 265                 270
   Val Glu Arg Met Gly His Met Leu Gln Arg Pro Leu Val Leu His Thr
           275                 280                 285
   Ser Ala Ala Ala Glu Leu Pro Arg Ala Asn Thr His Ile
       290                 295                 300

### (2) INFORMATION ZU SEQ ID NO: 3:

(i) SEQUENZ CHARAKTERISTIKA:
   (A) LÄNGE: 2627 Basenpaare
   (B) ART: Nukleinsäure
   (C) STRANGFORM: Doppel
   (D) TOPOLOGIE: linear
(ii) ART DES MOLEKÜLS: cDNS zu mRNS
(iii) HYPOTHETISCH: NEIN
(iii) ANTISENSE: NEIN
(vi) URSPRÜNLICHE HERKUNFT:
   (A) ORGANISMUS: Ashbya gossypii
(ix) MERKMALE:
   (A) NAME/SCHLÜSSEL: 5'UTR
   (B) LAGE: 1..450
(ix) MERKMALE:
   (A) NAME/SCHLÜSSEL: CDS
   (B) LAGE: 451..2280
(ix) MERKMALE:
   (A) NAME/SCHLÜSSEL: 3'UTR
   (B) LAGE: 2281..2627
(xi) SEQUENZBESCHREIBUNG: SEQ ID NO: 3:
   CTGCAGGACA ATTTAAATTA CGATTACACG CGGCAGCCTT CTTGGTGCGA CAGGATTTTG      60
   TACAAGAATG ACCCCAAGCG GGTAAGAGTT CATAGGTATG CCTCGATTGA TAGACGTTCC     120
   ATTTTGAATT ATACTGATCA CGAACCCGTA ACGCTCGATG TCAGCGTTTC ATGCCATACA     180
   CAATTTGTCC CAATGGCTAT GCAGAATATT TCCCCACAGA GCACCATGGA AATGTATGTG     240
   GGAGACGTCA CAGATATACT ACTGATGTTG TTCTCCAGAG TATACTACGC CCCTACCATA     300
   TTCGATCTTG TGGTATTGAC GATATTCCTC TGTTTGGTTT TACTGGCACT ATTCCGTTTG     360
   ACGGTATAGC GCTATTCGTT CATAGTGACA CATGCGGCAC TAGCTATTCA GCGAATCCTT     420
   TATAAACTGC TACTTAACGT TCGTAACACC ATG CTC AAA GGC GTT CCT GGC CTT      474
                                    Met Leu Lys Gly Val Pro Gly Leu
                                     1               5
   CTT TTT AAG GAG ACG CAA CGT CAT CTG AAA CCC AGG CTG GTT AGG ATT       522
   Leu Phe Lys Glu Thr Gln Arg His Leu Lys Pro Arg Leu Val Arg Ile
        10                  15                  20
   ATG GAA AAC ACA TCG CAG GAT GAG AGT CGC AAA AGA CAG GTC GCT TCG       570
   Met Glu Asn Thr Ser Gln Asp Glu Ser Arg Lys Arg Gln Val Ala Ser
    25                  30                  35                  40
   AAC TTG AGC AGC GAT GCC GAT GAG GGC TCG CCG GCA GTT ACG AGG CCG        618
   Asn Leu Ser Ser Asp Ala Asp Glu Gly Ser Pro Ala Val Thr Arg Pro
                       45                  50                  55
   GTT AAA ATC ACC AAA CGC CTC AGG AAG AAG AAC CTC GGG ACA GGC GAG        666
   Val Lys Ile Thr Lys Arg Leu Arg Lys Lys Asn Leu Gly Thr Gly Glu
                   60                  65                  70
   CTA CGG GAC AAA GCA GGA TTC AAG TTG AAG GTG CAA GAC GTG AGC AAA        714
   Leu Arg Asp Lys Ala Gly Phe Lys Leu Lys Val Gln Asp Val Ser Lys
               75                  80                  85
   AAC CGT CAC AGA CAG GTC GAT CCG GAA TAC GAA GTC GTG GTA GAT GGC        762
   Asn Arg His Arg Gln Val Asp Pro Glu Tyr Glu Val Val Val Asp Gly
           90                  95                  100
   CCG ATG CGC AAG ATC AAA CCG TAT TTC TTC ACA TAC AAG ACT TTC TGC        810
   Pro Met Arg Lys Ile Lys Pro Tyr Phe Phe Thr Tyr Lys Thr Phe Cys
   105                 110                 115                 120
   AAG GAG CGC TGG AGA GAT CGG AAG TTG CTT GAT GTG TTT GTG GAT GAA        858
   Lys Glu Arg Trp Arg Asp Arg Lys Leu Leu Asp Val Phe Val Asp Glu
                       125                 130                 135
   TTT CGG GAC CGC GAT AGG CCT TAC TAC GAG AAA GTC ATC GGT TCG GGT        906
   Phe Arg Asp Arg Asp Arg Pro Tyr Tyr Glu Lys Val Ile Gly Ser Gly
                   140                 145                 150
   GGT GTG CTC CTG AAC GGT AAG TCA TCG ACG TTA GAT AGC GTA TTG CGT        954
   Gly Val Leu Leu Asn Gly Lys Ser Ser Thr Leu Asp Ser Val Leu Arg
               155                 160                 165
   AAT GGA GAC CTC ATT TCG CAC GAG CTG CAC CGT CAT GAG CCA CCG GTC       1002
   Asn Gly Asp Leu Ile Ser His Glu Leu His Arg His Glu Pro Pro Val
           170                 175                 180
   TCC TCT AGG CCG ATT AGG ACG GTG TAC GAA GAT GAT GAC ATC CTG GTG       1050
   Ser Ser Arg Pro Ile Arg Thr Val Tyr Glu Asp Asp Asp Ile Leu Val
   185                 190                 195                 200
   ATT GAC AAG CCC AGC GGG ATT CCA GCC CAT CCC ACC GGG CGT TAC CGC       1098
   Ile Asp Lys Pro Ser Gly Ile Pro Ala His Pro Thr Gly Arg Tyr Arg
                       205                 210                 215
   TTC AAC TCC ATT ACG AAA ATA CTT GAA AAA CAG CTT GGA TAC ACT GTT       1146
   Phe Asn Ser Ile Thr Lys Ile Leu Glu Lys Gln Leu Gly Tyr Thr Val
                   220                 225                 230
   CAT CCA TGT AAC CGA CTG GAC CGC CTA ACC AGT GGC CTA ATG TTC TTG       1194
   His Pro Cys Asn Arg Leu Asp Arg Leu Thr Ser Gly Leu Met Phe Leu
               235                 240                 245
   GCA AAA ACT CCA AAG GGA GCC GAT GAG ATG GGT GAT CAG ATG AAG GCG       1242
   Ala Lys Thr Pro Lys Gly Ala Asp Glu Met Gly Asp Gln Met Lys Ala
           250                 255                 260
   CGC GAA GTG AAG AAA GAA TAT GTT GCC CGG GTT GTT GGG GAA TTT CCT       1290
   Arg Glu Val Lys Lys Glu Tyr Val Ala Arg Val Val Gly Glu Phe Pro
   265                 270                 275                 280
   ATA GGT GAG ATA GTT GTG GAT ATG CCA CTG AAG ACT ATA GAG CCG AAG      1338
   Ile Gly Glu Ile Val Val Asp Met Pro Leu Lys Thr Ile Glu Pro Lys
                   285                 290                 295
   CTT GCC CTA AAC ATG GTT TGC GAC CCG GAA GAC GAA GCG GGC AAG GGC      1386
   Leu Ala Leu Asn Met Val Cys Asp Pro Glu Asp Glu Ala Gly Lys Gly
               300                 305                 310
   GCT AAG ACG CAG TTC AAA AGA ATC AGC TAC GAT GGA CAA ACG AGC ATA      1434
   Ala Lys Thr Gln Phe Lys Arg Ile Ser Tyr Asp Gly Gln Thr Ser Ile
           315                 320                 325
   GTC AAG TGC CAA CCG TAC ACG GGC CGG ACG CAT CAG ATC CGT GTT CAC      1482
   Val Lys Cys Gln Pro Tyr Thr Gly Arg Thr His Gln Ile Arg Val His
       330                 335                 340
   TTG CAA TAC CTG GGC TTC CCA ATT GCC AAC GAT CCG ATT TAT TCC AAT      1530
   Leu Gln Tyr Leu Gly Phe Pro Ile Ala Asn Asp Pro Ile Tyr Ser Asn
   345                 350                 355                 360
   CCG CAC ATA TGG GGC CCA AGT CTG GGC AAG GAA TGC AAA GCA GAC TAC      1578
   Pro His Ile Trp Gly Pro Ser Leu Gly Lys Glu Cys Lys Ala Asp Tyr
                   365                 370                 375
   AAG GAG GTC ATC CAA AAA CTA AAC GAA ATT GGT AAG ACT AAA TCT GCG      1626
   Lys Glu Val Ile Gln Lys Leu Asn Glu Ile Gly Lys Thr Lys Ser Ala
               380                 385                 390
   GAA AGT TGG TAC CAT TCT GAT TCC CAA GGT GAA GTT TTC AAA GGG GAA      1674
   Glu Ser Trp Tyr His Ser Asp Ser Gln Gly Glu Val Phe Lys Gly Glu
           395                 400                 405
   CAA TGC GAT GAA TGT GGC ACC GAA CTG TAC ACT GAC CCG GGC CCG AAT      1722
   Gln Cys Asp Glu Cys Gly Thr Glu Leu Tyr Thr Asp Pro Gly Pro Asn
       410                 415                 420
   GAT CTT GAC TTA TGG TTG CAT GCA TAT CGG TAT GAA TCC ACT GAA CTG      1770
   Asp Leu Asp Leu Trp Leu His Ala Tyr Arg Tyr Glu Ser Thr Glu Leu
   425                 430                 435                 440
   GAT GAG AAC GGT GCT AAA AAG CGG AGT TAC TCT ACT GCG TTT CCT GAG      1818
   Asp Glu Asn Gly Ala Lys Lys Arg Ser Tyr Ser Thr Ala Phe Pro Glu
                   445                 450                 455
   TGG GCT CTT GAG CAG CAC GGC GAC TTC ATG CGG CTT GCC ATC GAA CAG      1866
   Trp Ala Leu Glu Gln His Gly Asp Phe Met Arg Leu Ala Ile Glu Gln
               460                 465                 470
   GCT AAG AAA TGC CCA CCC GCG AAG ACA TCA TTT AGC GTT GGT GCC GTG      1914
   Ala Lys Lys Cys Pro Pro Ala Lys Thr Ser Phe Ser Val Gly Ala Val
           475                 480                 485
   TTA GTT AAT GGG ACC GAG ATT TTG GCC ACT GGT TAC TCA CGG GAG CTG      1962
   Leu Val Asn Gly Thr Glu Ile Leu Ala Thr Gly Tyr Ser Arg Glu Leu
       490                 495                 500
   GAA GGC AAC ACG CAC GCT GAA CAA TGT GCA CTT CAA AAA TAT TTT GAA      2010
   Glu Gly Asn Thr His Ala Glu Gln Cys Ala Leu Gln Lys Tyr Phe Glu
   505                 510                 515                 520
   CAA CAT AAA ACC GAC AAG GTT CCT ATT GGT ACA GTA ATA TAC ACG ACT        2058
   Gln His Lys Thr Asp Lys Val Pro Ile Gly Thr Val Ile Tyr Thr Thr
                   525                 530                 535
   ATG GAG CCT TGT TCT CTC CGT CTC AGT GGT AAT AAA CCG TGT GTT GAG        2106
   Met Glu Pro Cys Ser Leu Arg Leu Ser Gly Asn Lys Pro Cys Val Glu
               540                 545                 550
   CGT ATA ATC TGC CAG CAG GGT AAT ATT ACT GCT GTT TTT GTT GGC GTA        2154
   Arg Ile Ile Cys Gln Gln Gly Asn Ile Thr Ala Val Phe Val Gly Val
           555                 560                 565
   CTT GAG CCA GAC AAC TTC GTG AAG AAC AAT ACA AGT CGT GCG CTA TTG        2202
   Leu Glu Pro Asp Asn Phe Val Lys Asn Asn Thr Ser Arg Ala Leu Leu
       570                 575                 580
   GAA CAA CAT GGT ATA GAC TAT ATT CTT GTC CCT GGG TTT CAA GAA GAA        2250
   Glu Gln His Gly Ile Asp Tyr Ile Leu Val Pro Gly Phe Gln Glu Glu
   585                 590                 595                 600
   TGT ACT GAA GCC GCA TTG AAG GGT CAT TGATTTTGCT GCGAATTGTA              2297
   Cys Thr Glu Ala Ala Leu Lys Gly His
                   605                 610
   GATGACTTAA AATATCGAGG CGTATAATTC GTCGCATTTT ATATAGTTAT CTATGTTTAC      2357
   ATGACTGTTT AAGCTTGATC TATATTTCTC AAGTGAATTG CCACATATGT TGGTACGGTA      2417
   ATAAATTAAT GAGGGAGTTT TGAAATTCGC AACCAATCTT ATATACGTTT GATGATATAA      2477
   ACGGATTGAG ATTCATTAAG CTACCTGATT TTCGCTGAAC TGTTTGTTAT AGGTTTTTAC      2537
   AGTAAGATAG TTCCTAAGTT TGTTTATTGT CCCCAGTCGG CCAATTGTTC CGGACTTATT      2597
   ATTATTACCA TTAGTGGTGT TAGTAGTATT                                       2627

### (2) INFORMATION ZU SEQ ID NO: 4:

(i) SEQUENZ CHARAKTERISTIKA:
   (A) LÄNGE: 609 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 4:
   Met Leu Lys Gly Val Pro Gly Leu Leu Phe Lys Glu Thr Gln Arg His
     1               5                  10                  15
   Leu Lys Pro Arg Leu Val Arg Ile Met Glu Asn Thr Ser Gln Asp Glu
                 20                  25                  30
   Ser Arg Lys Arg Gln Val Ala Ser Asn Leu Ser Ser Asp Ala Asp Glu
             35                  40                  45
   Gly Ser Pro Ala Val Thr Arg Pro Val Lys Ile Thr Lys Arg Leu Arg
         50                  55                  60
   Lys Lys Asn Leu Gly Thr Gly Glu Leu Arg Asp Lys Ala Gly Phe Lys
    65                  70                  75                  80
   Leu Lys Val Gln Asp Val Ser Lys Asn Arg His Arg Gln Val Asp Pro
                    85                  90                  95
   Glu Tyr Glu Val Val Val Asp Gly Pro Met Arg Lys Ile Lys Pro Tyr
               100                 105                 110
   Phe Phe Thr Tyr Lys Thr Phe Cys Lys Glu Arg Trp Arg Asp Arg Lys
           115                 120                 125
   Leu Leu Asp Val Phe Val Asp Glu Phe Arg Asp Arg Asp Arg Pro Tyr
       130                 135                 140
   Tyr Glu Lys Val Ile Gly Ser Gly Gly Val Leu Leu Asn Gly Lys Ser
   145                 150                 155                 160
   Ser Thr Leu Asp Ser Val Leu Arg Asn Gly Asp Leu Ile Ser His Glu
                   165                 170                 175
   Leu His Arg His Glu Pro Pro Val Ser Ser Arg Pro Ile Arg Thr Val
               180                 185                 190
   Tyr Glu Asp Asp Asp Ile Leu Val Ile Asp Lys Pro Ser Gly Ile Pro
           195                 200                 205
   Ala His Pro Thr Gly Arg Tyr Arg Phe Asn Ser Ile Thr Lys Ile Leu
       210                 215                 220
   Glu Lys Gln Leu Gly Tyr Thr Val His Pro Cys Asn Arg Leu Asp Arg
   225                 230                 235                 240
   Leu Thr Ser Gly Leu Met Phe Leu Ala Lys Thr Pro Lys Gly Ala Asp
                   245                 250                 255
   Glu Met Gly Asp Gln Met Lys Ala Arg Glu Val Lys Lys Glu Tyr Val
               260                 265                 270
   Ala Arg Val Val Gly Glu Phe Pro Ile Gly Glu Ile Val Val Asp Met
           275                 280                 285
   Pro Leu Lys Thr Ile Glu Pro Lys Leu Ala Leu Asn Met Val Cys Asp
       290                 295                 300
   Pro Glu Asp Glu Ala Gly Lys Gly Ala Lys Thr Gln Phe Lys Arg Ile
   305                 310                 315                 320
   Ser Tyr Asp Gly Gln Thr Ser Ile Val Lys Cys Gln Pro Tyr Thr Gly
                   325                 330                 335
   Arg Thr His Gln Ile Arg Val His Leu Gln Tyr Leu Gly Phe Pro Ile
               340                 345                 350
   Ala Asn Asp Pro Ile Tyr Ser Asn Pro His Ile Trp Gly Pro Ser Leu
           355                 360                 365
   Gly Lys Glu Cys Lys Ala Asp Tyr Lys Glu Val Ile Gln Lys Leu Asn
       370                 375                 380
   Glu Ile Gly Lys Thr Lys Ser Ala Glu Ser Trp Tyr His Ser Asp Ser
   385                 390                 395                 400
   Gln Gly Glu Val Phe Lys Gly Glu Gln Cys Asp Glu Cys Gly Thr Glu
                   405                 410                 415
   Leu Tyr Thr Asp Pro Gly Pro Asn Asp Leu Asp Leu Trp Leu His Ala
               420                 425                 430
   Tyr Arg Tyr Glu Ser Thr Glu Leu Asp Glu Asn Gly Ala Lys Lys Arg
           435                 440                 445
   Ser Tyr Ser Thr Ala Phe Pro Glu Trp Ala Leu Glu Gln His Gly Asp
       450                 455                 460
   Phe Met Arg Leu Ala Ile Glu Gln Ala Lys Lys Cys Pro Pro Ala Lys
   465                 470                 475                 480
   Thr Ser Phe Ser Val Gly Ala Val Leu Val Asn Gly Thr Glu Ile Leu
                   485                 490                 495
   Ala Thr Gly Tyr Ser Arg Glu Leu Glu Gly Asn Thr His Ala Glu Gln
               500                 505                 510
   Cys Ala Leu Gln Lys Tyr Phe Glu Gln His Lys Thr Asp Lys Val Pro
           515                 520                 525
   Ile Gly Thr Val Ile Tyr Thr Thr Met Glu Pro Cys Ser Leu Arg Leu
       530                 535                 540
   Ser Gly Asn Lys Pro Cys Val Glu Arg Ile Ile Cys Gln Gln Gly Asn
   545                 550                 555                 560
   Ile Thr Ala Val Phe Val Gly Val Leu Glu Pro Asp Asn Phe Val Lys
                   565                 570                 575
   Asn Asn Thr Ser Arg Ala Leu Leu Glu Gln His Gly Ile Asp Tyr Ile
               580                 585                 590
   Leu Val Pro Gly Phe Gln Glu Glu Cys Thr Glu Ala Ala Leu Lys Gly
           595                 600                 605
   His

### (2) INFORMATION ZU SEQ ID NO: 5:

(i) SEQUENZ CHARAKTERISTIKA:
   (A) LÄNGE: 1082 Basenpaare
   (B) ART: Nukleinsäure
   (C) STRANGFORM: Doppel
   (D) TOPOLOGIE: linear
(ii) ART DES MOLEKÜLS: cDNS zu mRNS
(iii) HYPOTHETISCH: NEIN
(iii) ANTISENSE: NEIN
(vi) URSPRÜNLICHE HERKUNFT:
   (A) ORGANISMUS: Ashbya gossypii
(ix) MERKMALE:
   (A) NAME/SCHLÜSSEL: 5'UTR
   (B) LAGE: 1..314
(ix) MERKMALE:
   (A) NAME/SCHLÜSSEL: CDS
   (B) LAGE: 315..953
(ix) MERKMALE:
   (A) NAME/SCHLÜSSEL: 3'UTR
   (B) LAGE: 954..1082
(xi) SEQUENZBESCHREIBUNG: SEQ ID NO: 5:
   CCCTTCTTGC ACGGTCGTTT CTGAAACTCT ACGATTATTG GAACAATGAG TAAGTCCTCA     60
   AATGTACCAC CTATCTGTAG TTTACTATCG GATTTACTGG CTAAGAGCTG ACCTGTTAGG    120
   CAAGTGAAAC ATATCACATC GCCAGCAGGT TGGGCTACCA AGGATAGTTG ATGACTTCCA    180
   TCACCTATAA AAGCGGCTTG AGTGCTTTTG CAATGATTCT GTTCACATGA TGGACAAGAA    240
   ATACGTACAA AAATTTCAAC GTTTTACAAG TTCCCAAGCT TAGTCAACTC ATCACCAACG    300
   ACAAACCAAG CAAC ATG ACA AGC CCA TGC ACT GAT ATC GGT ACC GCT ATA      350
                   Met Thr Ser Pro Cys Thr Asp Ile Gly Thr Ala Ile
                    1               5                  10
   GAG CAG TTC AAG CAA AAT AAG ATG ATC ATC GTC ATG GAC CAC ATC TCG      398
   Glu Gln Phe Lys Gln Asn Lys Met Ile Ile Val Met Asp His Ile Ser
            15                  20                  25
   AGA GAA AAC GAG GCC GAT CTA ATA TGT GCA GCA GCG CAC ATG ACT GCC      446
   Arg Glu Asn Glu Ala Asp Leu Ile Cys Ala Ala Ala His Met Thr Ala
        30                  35                  40
   GAG CAA ATG GCA TTT ATG ATT CGG TAT TCC TCG GGC TAC GTT TGC GCT      494
   Glu Gln Met Ala Phe Met Ile Arg Tyr Ser Ser Gly Tyr Val Cys Ala
    45                  50                  55                  60
   CCA ATG ACC AAT GCG ATT GCC GAT AAG CTA GAC CTA CCG CTC ATG AAC      542
   Pro Met Thr Asn Ala Ile Ala Asp Lys Leu Asp Leu Pro Leu Met Asn
                    65                  70                  75
   ACA TTG AAA TGC AAG GCT TTC TCC GAT GAC AGA CAC AGC ACT GCG TAT      590
   Thr Leu Lys Cys Lys Ala Phe Ser Asp Asp Arg His Ser Thr Ala Tyr
                80                  85                  90
   ACA ATC ACC TGT GAC TAT GCG CAC GGG ACG ACG ACA GGT ATC TCC GCA      638
   Thr Ile Thr Cys Asp Tyr Ala His Gly Thr Thr Thr Gly Ile Ser Ala
            95                 100                 105
   CGT GAC CGG GCG TTG ACC GTG AAT CAG TTG GCG AAC CCG GAG TCC AAG      686
   Arg Asp Arg Ala Leu Thr Val Asn Gln Leu Ala Asn Pro Glu Ser Lys
       110                 115                 120
   GCT ACC GAC TTC ACG AAG CCA GGC CAC ATT GTG CCA TTG CGT GCC CGT      734
   Ala Thr Asp Phe Thr Lys Pro Gly His Ile Val Pro Leu Arg Ala Arg
   125                 130                 135                 140
   GAC GGC GGC GTG CTC GAG CGT GAC GGG CAC ACC GAA GCG GCG CTC GAC      782
   Asp Gly Gly Val Leu Glu Arg Asp Gly His Thr Glu Ala Ala Leu Asp
                   145                 150                 155
   TTG TGC AGA CTA GCG GGT GTG CCA GAG GTC GCT GCT ATT TGT GAA TTA      830
   Leu Cys Arg Leu Ala Gly Val Pro Glu Val Ala Ala Ile Cys Glu Leu
               160                 165                 170
   GTA AGC GAA AGG GAC GTC GGG CTG ATG ATG ACT TTG GAT GAG TGT ATA      878
   Val Ser Glu Arg Asp Val Gly Leu Met Met Thr Leu Asp Glu Cys Ile
           175                 180                 185
   GAA TTC AGC AAG AAG CAC GGT CTT GCC CTC ATC ACC GTG CAT GAC CTG      926
   Glu Phe Ser Lys Lys His Gly Leu Ala Leu Ile Thr Val His Asp Leu
       190                 195                 200
   AAG GCT GCA GTT GCC GCC AAG CAG TAGACGGCAA CGAGTTCTTT AAGTCGGTGT     980
   Lys Ala Ala Val Ala Ala Lys Gln
   205                 210
   TCATTTATGT AATATACCAT TTCATCGAAA AAGTCAAATG GTATGAACTA GATTTATCAA   1040
   TAGTATCTAA GAGTTATGGT ATTCGCAAAA GCTTATCGAT AC                      1082

### (2) INFORMATION ZU SEQ ID NO: 6:

(i) SEQUENZ CHARAKTERISTIKA:
   (A) LÄNGE: 212 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 6:
   Met Thr Ser Pro Cys Thr Asp Ile Gly Thr Ala Ile Glu Gln Phe Lys
    1               5                  10                  15
   Gln Asn Lys Met Ile Ile Val Met Asp His Ile Ser Arg Glu Asn Glu
               20                  25                  30
   Ala Asp Leu Ile Cys Ala Ala Ala His Met Thr Ala Glu Gln Met Ala
           35                  40                  45
   Phe Met Ile Arg Tyr Ser Ser Gly Tyr Val Cys Ala Pro Met Thr Asn
       50                  55                  60
   Ala Ile Ala Asp Lys Leu Asp Leu Pro Leu Met Asn Thr Leu Lys Cys
   65                  70                  75                  80
   Lys Ala Phe Ser Asp Asp Arg His Ser Thr Ala Tyr Thr Ile Thr Cys
                   85                  90                  95
   Asp Tyr Ala His Gly Thr Thr Thr Gly Ile Ser Ala Arg Asp Arg Ala
               100                 105                 110
   Leu Thr Val Asn Gln Leu Ala Asn Pro Glu Ser Lys Ala Thr Asp Phe
           115                 120                 125
   Thr Lys Pro Gly His Ile Val Pro Leu Arg Ala Arg Asp Gly Gly Val
       130                 135                 140
   Leu Glu Arg Asp Gly His Thr Glu Ala Ala Leu Asp Leu Cys Arg Leu
   145                 150                 155                 160
   Ala Gly Val Pro Glu Val Ala Ala Ile Cys Glu Leu Val Ser Glu Arg
                   165                 170                 175
   Asp Val Gly Leu Met Met Thr Leu Asp Glu Cys Ile Glu Phe Ser Lys
               180                 185                 190
   Lys His Gly Leu Ala Leu Ile Thr Val His Asp Leu Lys Ala Ala Val
           195                 200                 205
   Ala Ala Lys Gln
       210

### (2) INFORMATION ZU SEQ ID NO: 7:

(i) SEQUENZ CHARAKTERISTIKA:
   (A) LÄNGE: 996 Basenpaare
   (B) ART: Nukleinsäure
   (C) STRANGFORM: Doppel
   (D) TOPOLOGIE: linear
(ii) ART DES MOLEKÜLS: cDNS zu mRNS
(iii) HYPOTHETISCH: NEIN
(iii) ANTISENSE: NEIN
(vi) URSPRÜNLICHE HERKUNFT:
   (A) ORGANISMUS: Ashbya gossypii
(ix) MERKMALE:
   (A) NAME/SCHLÜSSEL: 5'UTR
   (B) LAGE: 1..270
(ix) MERKMALE:
   (A) NAME/SCHLÜSSEL: CDS
   (B) LAGE: 271..789
(ix) MERKMALE:
   (A) NAME/SCHLÜSSEL: 3'UTR
   (B) LAGE: 790..996
(xi) SEQUENZBESCHREIBUNG: SEQ ID NO: 7:
   TGGTATAATG ATACAGGAAG TGAAAATCCG AAAGGTTCAG ACGATGAAAA GAGTTTGAGA      60
   CGCATCAATG ATCAGCTTTG AGCTATATGT AAGTCTATTA ATTGATTACT AATAGCAATT     120
   TATGGTATCC TCTGTTCTGC ATATCGACGG TTCTCACGTG ATGATCAGCT TGAGGCTTCG     180
   CGGATAAAGT TCCATCGATT ACTATAAAAC CATCACATTA AACGTTCACT ATAGGCATAC     240
   ACACAGACTA AGTTCAAGTT AGCAGTGACA ATG ATT AAG GGA TTA GGC GAA GTT      294
                                    Met Ile Lys Gly Leu Gly Glu Val
                                      1               5
   GAT CAA ACC TAC GAT GCG AGC TCT GTC GAG GTT GGC ATT GTC CAC GCG       342
   Asp Gln Thr Tyr Asp Ala Ser Ser Val Glu Val Gly Ile Val His Ala
        10                  15                  20
   AGA TGG AAC AAG ACT GTC ATT GAC GCT CTC GAC CAA GGT GCA ATT GAG       390
   Arg Trp Asn Lys Thr Val Ile Asp Ala Leu Asp Gln Gly Ala Ile Glu
    25                  30                  35                  40
   AAA CTG CTT GCT ATG GGA GTG AAG GAG AAG AAT ATC ACT GTA AGC ACC       438
   Lys Leu Leu Ala Met Gly Val Lys Glu Lys Asn Ile Thr Val Ser Thr
                    45                  50                  55
   GTT CCA GGT GCG TTT GAA CTA CCA TTT GGC ACT CAG CGG TTT GCC GAG       486
   Val Pro Gly Ala Phe Glu Leu Pro Phe Gly Thr Gln Arg Phe Ala Glu
                60                  65                  70
   CTG ACC AAG GCA AGT GGC AAG CAT TTG GAC GTG GTC ATC CCA ATT GGA       534
   Leu Thr Lys Ala Ser Gly Lys His Leu Asp Val Val Ile Pro Ile Gly
            75                  80                  85
   GTC CTG ATC AAA GGC GAC TCA ATG CAC TTT GAA TAT ATA TCA GAC TCT       582
   Val Leu Ile Lys Gly Asp Ser Met His Phe Glu Tyr Ile Ser Asp Ser
        90                  95                 100
   GTG ACT CAT GCC TTA ATG AAC CTA CAG AAG AAG ATT CGT CTT CCT GTC       630
   Val Thr His Ala Leu Met Asn Leu Gln Lys Lys Ile Arg Leu Pro Val
   105                 110                 115                 120
   ATT TTT GGT TTG CTA ACG TGT CTA ACA GAG GAA CAA GCG TTG ACA CGT       678
   Ile Phe Gly Leu Leu Thr Cys Leu Thr Glu Glu Gln Ala Leu Thr Arg
                   125                 130                 135
   GCA GGC CTC GGT GAA TCT GAA GGC AAG CAC AAC CAC GGT GAA GAC TGG       726
   Ala Gly Leu Gly Glu Ser Glu Gly Lys His Asn His Gly Glu Asp Trp
               140                 145                 150
   GGT GCT GCT GCC GTG GAG ATG GCT GTA AAG TTT GGC CCA CGC GCC GAA       774
   Gly Ala Ala Ala Val Glu Met Ala Val Lys Phe Gly Pro Arg Ala Glu
           155                 160                 165
   CAA ATG AAG AAG TGAATATTAA AAAATCACTA CTTAAAATTA ACGTTTTTAT           826
   Gln Met Lys Lys
       170
   TATGTCTATA TCAAATTCTT ACGTGATAAC TTTTGATTTC GCTTCCTGGA TTGGCGCAAG     886
   GCCTCCCTGT GTCGCAGTTT TTGTTCACGG GTCCACACAG CTCTGTTTTC CCAGAACATA     946
   TCCTCCCAGC CGGCGAACCG GTTAGACGCT TCTGCTGGCG TTCTTATTTT               996

### (2) INFORMATION ZU SEQ ID NO: 8:

(i) SEQUENZ CHARAKTERISTIKA:
   (A) LÄNGE: 172 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 8:
   Met Ile Lys Gly Leu Gly Glu Val Asp Gln Thr Tyr Asp Ala Ser Ser
    1               5                  10                  15
   Val Glu Val Gly Ile Val His Ala Arg Trp Asn Lys Thr Val Ile Asp
               20                  25                  30
   Ala Leu Asp Gln Gly Ala Ile Glu Lys Leu Leu Ala Met Gly Val Lys
           35                  40                  45
   Glu Lys Asn Ile Thr Val Ser Thr Val Pro Gly Ala Phe Glu Leu Pro
       50                  55                  60
   Phe Gly Thr Gln Arg Phe Ala Glu Leu Thr Lys Ala Ser Gly Lys His
   65                  70                  75                  80
   Leu Asp Val Val Ile Pro Ile Gly Val Leu Ile Lys Gly Asp Ser Met
                   85                  90                  95
   His Phe Glu Tyr Ile Ser Asp Ser Val Thr His Ala Leu Met Asn Leu
               100                 105                 110
   Gln Lys Lys Ile Arg Leu Pro Val Ile Phe Gly Leu Leu Thr Cys Leu
           115                 120                 125
   Thr Glu Glu Gln Ala Leu Thr Arg Ala Gly Leu Gly Glu Ser Glu Gly
       130                 135                 140
   Lys His Asn His Gly Glu Asp Trp Gly Ala Ala Ala Val Glu Met Ala
   145                 150                 155                 160
   Val Lys Phe Gly Pro Arg Ala Glu Gln Met Lys Lys
                   165                 170

### (2) INFORMATION ZU SEQ ID NO: 9:

(i) SEQUENZ CHARAKTERISTIKA:
   (A) LÄNGE: 1511 Basenpaare
   (B) ART: Nukleinsäure
   (C) STRANGFORM: Doppel
   (D) TOPOLOGIE: linear
(ii) ART DES MOLEKÜLS: cDNS zu mRNS
(iii) HYPOTHETISCH: NEIN
(iii) ANTISENSE: NEIN
(vi) URSPRÜNLICHE HERKUNFT:
   (A) ORGANISMUS: Ashbya gossypii
(ix) MERKMALE:
   (A) NAME/SCHLÜSSEL: 5'UTR
   (B) LAGE: 1..524
(ix) MERKMALE:
   (A) NAME/SCHLÜSSEL: CDS
   (B) LAGE: 525..1232
(ix) MERKMALE:
   (A) NAME/SCHLÜSSEL: 3'UTR
   (B) LAGE: 1233..1511
(xi) SEQUENZBESCHREIBUNG: SEQ ID NO: 9:
   TGTATTCAAC CTGGAGGATA ACGAAATTTC CATGGCGCGG GCGATACCAA CCCACAGGAG      60
   CCAGATATAA GACCAATCCC GGCGGGTGTG CCAGCCGCCA TCAGAGACAG CGGGCCAGCA     120
   AGGCATGTGA AGTCAAAAGG CGCCAGCTCC TTATCCGCTC CCGCACAAGC AGGACCGGCA     180
   TATCCCGATG AGCGCGCCAG CACCCAGACG CTACACCACC ATTCGAAGTA GACTTTAAAA     240
   GAGCGCTTTC CAGCTTCTCA GGCAGTTAGC TCTACGACAA AGGAACCAAG TGATTTTCCC     300
   GATAGACGCG ACTTGCTCAA CGATGTTTCT GTGACCAGCG CAAGGAGAGA TAGTCCTAAA     360
   GTATAATCAG ATAGTTAGTC GTATCTTCTA GTTTTATTAG TCAGCTACAT GGCGAACCGC     420
   CATTTCCTTA TGCATGTCTT ACGAGTTTAA AAAGCTCGCG GTAGCAGAAA AGAAGATGCA     480
   TAGATGGCAT ACCGAAGCCT ATATCGCCCA TAGAAGTTGA TAGG ATG TTT ACC GGT      536
                                                    Met Phe Thr Gly
                                                      1
   ATA GTG GAA CAC ATT GGC ACT GTT GCT GAG TAC TTG GAG AAC GAT GCC       584
   Ile Val Glu His Ile Gly Thr Val Ala Glu Tyr Leu Glu Asn Asp Ala
     5                  10                  15                  20
   AGC GAG GCA GGC GGC AAC GGT GTG TCA GTC CTT ATC AAG GAT GCG GCT       632
   Ser Glu Ala Gly Gly Asn Gly Val Ser Val Leu Ile Lys Asp Ala Ala
                    25                  30                  35
   CCG ATA CTG GCG GAT TGC CAC ATC GGT GAC TCG ATT GCA TGC AAT GGT       680
   Pro Ile Leu Ala Asp Cys His Ile Gly Asp Ser Ile Ala Cys Asn Gly
                40                  45                  50
   ATC TGC CTG ACG GTG ACG GAG TTC ACG GCC GAT AGC TTC AAG GTC GGG       728
   Ile Cys Leu Thr Val Thr Glu Phe Thr Ala Asp Ser Phe Lys Val Gly
            55                  60                  65
   ATC GCA CCA GAA ACA GTT TAT CGG ACG GAA GTC AGC AGC TGG AAA GCT       776
   Ile Ala Pro Glu Thr Val Tyr Arg Thr Glu Val Ser Ser Trp Lys Ala
        70                  75                  80
   GGC TCC AAG ATC AAC CTA GAA AGG GCC ATC TCG GAC GAC AGG CGC TAC       824
   Gly Ser Lys Ile Asn Leu Glu Arg Ala Ile Ser Asp Asp Arg Arg Tyr
    85                  90                  95                 100
   GGC GGG CAC TAC GTG CAG GGC CAC GTC GAC TCG GTG GCC TCT ATT GTA       872
   Gly Gly His Tyr Val Gln Gly His Val Asp Ser Val Ala Ser Ile Val
                   105                 110                 115
   TCC AGA GAG CAC GAC GGG AAC TCT ATC AAC TTT AAG TTT AAA CTG CGC       920
   Ser Arg Glu His Asp Gly Asn Ser Ile Asn Phe Lys Phe Lys Leu Arg
               120                 125                 130
   GAT CAA GAG TAC GAG AAG TAC GTA GTA GAA AAG GGT TTT GTG GCG ATC       968
   Asp Gln Glu Tyr Glu Lys Tyr Val Val Glu Lys Gly Phe Val Ala Ile
           135                 140                 145
   GAC GGT GTG TCG CTG ACT GTA AGC AAG ATG GAT CCA GAT GGC TGT TTC      1016
   Asp Gly Val Ser Leu Thr Val Ser Lys Met Asp Pro Asp Gly Cys Phe
       150                 155                 160
   TAC ATC TCG ATG ATT GCA CAC ACG CAG ACC GCT GTA GCC CTT CCA CTG      1064
   Tyr Ile Ser Met Ile Ala His Thr Gln Thr Ala Val Ala Leu Pro Leu
   165                 170                 175                 180
   AAG CCG GAC GGT GCC CTC GTG AAC ATA GAA ACG GAT GTT AAC GGC AAG      1112
   Lys Pro Asp Gly Ala Leu Val Asn Ile Glu Thr Asp Val Asn Gly Lys
                   185                 190                 195
   CTA GTA GAG AAG CAG GTT GCA CAG TAC CTG AAT GCG CAG CTG GAA GGT      1160
   Leu Val Glu Lys Gln Val Ala Gln Tyr Leu Asn Ala Gln Leu Glu Gly
               200                 205                 210
   GAG AGC TCG CCA TTG CAG CGC GTG CTC GAA AGG ATT ATT GAA TCC AAG      1208
   Glu Ser Ser Pro Leu Gln Arg Val Leu Glu Arg Ile Ile Glu Ser Lys
           215                 220                 225
   CTT GCT AGC ATC TCA AAT AAG TGATTATATT ATCTTGGGTG CTGTATATCT         1259
   Leu Ala Ser Ile Ser Asn Lys
       230                 235
   TATGTATGTC TTACGACTGT GAATCAGAGG GGTGGCAGCT GGAACACCAG CGACACACCT    1319
   TCGTCTCCCG CGGTGATCAG CCTTCTGTTT TCCTCAAGTA GTACAAAGTC TAGGACACCC    1379
   TGTTGTGGCC AACGCAAACA TGGAGCTGCT GCCCGTTACG CACGTCGAAC TCGTAGACCT    1439
   TGCCGTCAAT GCACGAGGCG AACAGGTGGA AACCGGTGGT CTTGTCAAAC CGCCAGCTTC    1499
   GTGACCGAGT CC                                                        1511

### (2) INFORMATION ZU SEQ ID NO: 10:

(i) SEQUENZ CHARAKTERISTIKA:
   (A) LÄNGE: 235 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 10:
   Met Phe Thr Gly Ile Val Glu His Ile Gly Thr Val Ala Glu Tyr Leu
     1               5                  10                  15
   Glu Asn Asp Ala Ser Glu Ala Gly Gly Asn Gly Val Ser Val Leu Ile
                20                  25                  30
   Lys Asp Ala Ala Pro Ile Leu Ala Asp Cys His Ile Gly Asp Ser Ile
            35                  40                  45
   Ala Cys Asn Gly Ile Cys Leu Thr Val Thr Glu Phe Thr Ala Asp Ser
        50                  55                  60
   Phe Lys Val Gly Ile Ala Pro Glu Thr Val Tyr Arg Thr Glu Val Ser
    65                  70                  75                  80
   Ser Trp Lys Ala Gly Ser Lys Ile Asn Leu Glu Arg Ala Ile Ser Asp
                    85                  90                  95
   Asp Arg Arg Tyr Gly Gly His Tyr Val Gln Gly His Val Asp Ser Val
               100                 105                 110
   Ala Ser Ile Val Ser Arg Glu His Asp Gly Asn Ser Ile Asn Phe Lys
           115                 120                 125
   Phe Lys Leu Arg Asp Gln Glu Tyr Glu Lys Tyr Val Val Glu Lys Gly
       130                 135                 140
   Phe Val Ala Ile Asp Gly Val Ser Leu Thr Val Ser Lys Met Asp Pro
   145                 150                 155                 160
   Asp Gly Cys Phe Tyr Ile Ser Met Ile Ala His Thr Gln Thr Ala Val
                   165                 170                 175
   Ala Leu Pro Leu Lys Pro Asp Gly Ala Leu Val Asn Ile Glu Thr Asp
               180                 185                 190
   Val Asn Gly Lys Leu Val Glu Lys Gln Val Ala Gln Tyr Leu Asn Ala
           195                 200                 205
   Gln Leu Glu Gly Glu Ser Ser Pro Leu Gln Arg Val Leu Glu Arg Ile
       210                 215                 220
   Ile Glu Ser Lys Leu Ala Ser Ile Ser Asn Lys
   225                 230                 235

### (2) INFORMATION ZU SEQ ID NO: 11:

(i) SEQUENZ CHARAKTERISTIKA:
   (A) LÄNGE: 1596 Basenpaare
   (B) ART: Nukleinsäure
   (C) STRANGFORM: Doppel
   (D) TOPOLOGIE: linear
(ii) ART DES MOLEKÜLS: cDNS zu mRNS
(iii) HYPOTHETISCH: NEIN
(iii) ANTISENSE: NEIN
(vi) URSPRÜNLICHE HERKUNFT:
   (A) ORGANISMUS: Ashbya gossypii
(ix) MERKMALE:
   (A) NAME/SCHLÜSSEL: 5'UTR
   (B) LAGE: 1..352
(ix) MERKMALE:
   (A) NAME/SCHLÜSSEL: CDS
   (B) LAGE: 353..1093
(ix) MERKMALE:
   (A) NAME/SCHLÜSSEL: 3'UTR
   (B) LAGE: 1094..1596
(xi) SEQUENZBESCHREIBUNG: SEQ ID NO: 11:
   AGAAGAAGCG CAGGCGCCAG TCCGAGCTGG AGGAGAACGA GGCGGCGCGG TTGACGAACA     60
   GCGCGCTGCC CATGGACGAT GCGGGTATAC AGACGGCGGG TATACAGACG GCGGGTGGTG    120
   CCGAGAGAGG CACCAGGCCG GCTTCCTCCA GCGATGCAAG GAAGAGAAGG GGACCAGAGG    180
   CGAAGTTCAA GCCATCTAAG GTACAGAAGC CCCAATTGAA GCGAACTGCA TCGTCCCGGG    240
   CGGATGAGAA CGAGTTCTCG ATATTATAGA GGCCCCCGTT TCGAGTGATT GGCGTCAAAA    300
   ACGGCTATCT GCCTTCGTCC GCCCCCACCA CCCTCGGGAA CACTGGCAAA CC ATG       355
                                                             Met
                                                               1
   GCG CTA ATA CCA CTT TCT CAA GAT CTG GCT GAT ATA CTA GCA CCG TAC      403
   Ala Leu Ile Pro Leu Ser Gln Asp Leu Ala Asp Ile Leu Ala Pro Tyr
               5                  10                  15
   TTA CCG ACA CCA CCG GAC TCA TCC GCA CGC CTG CCG TTT GTC ACG CTG      451
   Leu Pro Thr Pro Pro Asp Ser Ser Ala Arg Leu Pro Phe Val Thr Leu
           20                  25                  30
   ACG TAT GCG CAG TCC CTA GAT GCT CGT ATC GCG AAG CAA AAG GGT GAA      499
   Thr Tyr Ala Gln Ser Leu Asp Ala Arg Ile Ala Lys Gln Lys Gly Glu
        35                  40                  45
   AGG ACG GTT ATT TCG CAT GAG GAG ACC AAG ACA ATG ACG CAT TAT CTA      547
   Arg Thr Val Ile Ser His Glu Glu Thr Lys Thr Met Thr His Tyr Leu
    50                  55                  60                  65
   CGC TAC CAT CAT AGC GGC ATC CTG ATT GGC TCG GGC ACA GCC CTT GCG      595
   Arg Tyr His His Ser Gly Ile Leu Ile Gly Ser Gly Thr Ala Leu Ala
                    70                  75                  80
   GAC GAC CCG GAT CTC AAT TGC CGG TGG ACA CCT GCA GCG GAC GGG GCG      643
   Asp Asp Pro Asp Leu Asn Cys Arg Trp Thr Pro Ala Ala Asp Gly Ala
                85                  90                  95
   GAT TGC ACC GAA CAG TCT TCA CCA CGA CCC ATT ATC TTG GAT GTT CGG      691
   Asp Cys Thr Glu Gln Ser Ser Pro Arg Pro Ile Ile Leu Asp Val Arg
           100                 105                 110
   GGC AGA TGG AGA TAC CGC GGG TCC AAA ATA GAG TAT CTG CAT AAC CTT      739
   Gly Arg Trp Arg Tyr Arg Gly Ser Lys Ile Glu Tyr Leu His Asn Leu
       115                 120                 125
   GGC AAG GGG AAG GCG CCC ATA GTG GTC ACG GGG GGT GAG CCG GAG GTC      787
   Gly Lys Gly Lys Ala Pro Ile Val Val Thr Gly Gly Glu Pro Glu Val
   130                 135                 140                 145
   CGC GAA CTA GGC GTC AGT TAC CTG CAG CTG GGT GTC GAC GAG GGT GGC      835
   Arg Glu Leu Gly Val Ser Tyr Leu Gln Leu Gly Val Asp Glu Gly Gly
                   150                 155                 160
   CGC TTG AAT TGG GGC GAG TTG TTT GAG CGA CTC TAT TCT GAG CAC CAC      883
   Arg Leu Asn Trp Gly Glu Leu Phe Glu Arg Leu Tyr Ser Glu His His
               165                 170                 175
   CTG GAA AGT GTC ATG GTC GAA GGC GGC GCG GAG GTG CTC AAC CAG CTG      931
   Leu Glu Ser Val Met Val Glu Gly Gly Ala Glu Val Leu Asn Gln Leu
           180                 185                 190
   CTG CTG CGC CCA GAT ATT GTG GAC AGT CTG GTG ATC ACG ATA GGA TCC      979
   Leu Leu Arg Pro Asp Ile Val Asp Ser Leu Val Ile Thr Ile Gly Ser
       195                 200                 205
   AAG TTC CTG GGC TCA CTA GGT GTT GCG GTC TCA CCA GCT GAG GAG GTG     1027
   Lys Phe Leu Gly Ser Leu Gly Val Ala Val Ser Pro Ala Glu Glu Val
   210                 215                 220                 225
   AAC CTA GAG CAT GTG AAC TGG TGG CAC GGA ACA AGT GAC AGT GTT TTG     1075
   Asn Leu Glu His Val Asn Trp Trp His Gly Thr Ser Asp Ser Val Leu
                   230                 235                 240
   TGC GGC CGG CTC GCA TAGCGGTTAT GACTGGTCTA CTAGTTAAAA CTATTTACTC     1130
   Cys Gly Arg Leu Ala
               245
   CTATACATAT TGCGTCACAT AGCGTTTATC CCCCTCGCCA ACCGCCTCGT GCCGTTGGAA   1190
   ACACGGCGGC CGGGGGACCT CAAGCGCTCC GCATCGACTA GTTTAATTTA CAAACAGATT   1250
   CTGTAACTTG CGTAACGGCC AGAGGTCTCT GACTTTCTGA TAATCTTCAC CACCTCACCT   1310
   CGCTTCAACC CCAGGTATAA TGCAACTTGG ATCCATCCTC TGGATTCTAG GTAACTGAGA   1370
   TTCCTTTAAC CTGTATCTCT TCAACAACTC CTTCTTTTCT TCGTCGCTGA GTTTGATATG   1430
   TTTTGGCACA AGCTCATGGT GCGTGATATT TACCACCAAA GCTGTTTCGT TGAAAGTCTC    1490
   AATTGTAGCA GGAGCGACGG AGGGAAGCAG TTTCAACGCG CTGGGCGTTA TGCCGTTCTG    1550
   ATATATGAAA ATACCCGTCT GGAAGTTCTT CTCGCCAATG TGGATC                   1596

### (2) INFORMATION ZU SEQ ID NO: 12:

(i) SEQUENZ CHARAKTERISTIKA:
   (A) LÄNGE: 246 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 12:
   Met Ala Leu Ile Pro Leu Ser Gln Asp Leu Ala Asp Ile Leu Ala Pro
    1               5                  10                  15
   Tyr Leu Pro Thr Pro Pro Asp Ser Ser Ala Arg Leu Pro Phe Val Thr
               20                  25                  30
   Leu Thr Tyr Ala Gln Ser Leu Asp Ala Arg Ile Ala Lys Gln Lys Gly
           35                  40                  45
   Glu Arg Thr Val Ile Ser His Glu Glu Thr Lys Thr Met Thr His Tyr
       50                  55                  60
   Leu Arg Tyr His His Ser Gly Ile Leu Ile Gly Ser Gly Thr Ala Leu
   65                  70                  75                  80
   Ala Asp Asp Pro Asp Leu Asn Cys Arg Trp Thr Pro Ala Ala Asp Gly
                   85                  90                  95
   Ala Asp Cys Thr Glu Gln Ser Ser Pro Arg Pro Ile Ile Leu Asp Val
               100                 105                 110
   Arg Gly Arg Trp Arg Tyr Arg Gly Ser Lys Ile Glu Tyr Leu His Asn
           115                 120                 125
   Leu Gly Lys Gly Lys Ala Pro Ile Val Val Thr Gly Gly Glu Pro Glu
       130                 135                 140
   Val Arg Glu Leu Gly Val Ser Tyr Leu Gln Leu Gly Val Asp Glu Gly
   145                 150                 155                 160
   Gly Arg Leu Asn Trp Gly Glu Leu Phe Glu Arg Leu Tyr Ser Glu His
                   165                 170                 175
   His Leu Glu Ser Val Met Val Glu Gly Gly Ala Glu Val Leu Asn Gln
               180                 185                 190
   Leu Leu Leu Arg Pro Asp Ile Val Asp Ser Leu Val Ile Thr Ile Gly
           195                 200                 205
   Ser Lys Phe Leu Gly Ser Leu Gly Val Ala Val Ser Pro Ala Glu Glu
       210                 215                 220
   Val Asn Leu Glu His Val Asn Trp Trp His Gly Thr Ser Asp Ser Val
   225                 230                 235                 240
   Leu Cys Gly Arg Leu Ala
                   245

## Beschreibung

Die vorliegende Erfindung betrifft die Gene für Riboflavin-Biosynthese in Pilzen, die damit codierten Proteine sowie gentechnische Verfahren zur Herstellung von Riboflavin unter Verwendung dieser Gene und Genprodukte.

Die Herstellung von Riboflavin durch Fermentation von Pilzen wie Eremothecium ashbyii oder Ashbya gossypii ist bekannt (The Merck Index, Windholz et al., eds. Merck & Co., Seite 1183, 1983).

In der EP 405370 sind Riboflavin-überproduzierende Bakterienstämme beschrieben, die durch Transformation der Riboflavin-Biosynthese-Gene aus Bacillus subtilis erhalten wurden.

Da die Genetik der Riboflavin-Biosynthese in Bakterien und Eukaryonten verschieden ist, sind die oben erwähnten Gene aus Bacillus subtilis nicht für ein rekombinantes Herstellverfahren für Riboflavin mit eukaryontischen Produktionsorganismen wie Ashbya gossypii geeignet.

In einer am 19.11.1992 beim Deutschen Patentamt eingereichten Patentanmeldung wurde die Klonierung der Riboflavin-Biosynthese Gene der Hefe Saccharomyces cerevisiae beschrieben.

Eine Klonierung der Ashbya gossypii Riboflavin-Biosynthese Gene unter Verwendung der S. cerevisiae RIB-Gene mit üblichen Hybridisierungsmethoden gelang jedoch nicht; offenbar war die Homologie der RIB-Gene aus S. cerevisiae und A. gossypii für eine Hybridisierung nicht groß genug.

Es bestand daher die Aufgabe, die Riboflavin-Biosynthese Gene aus einem Eukaryonten zu isolieren, um damit ein rekombinantes Herstellverfahren für Riboflavin in einem eukaryontischen Produktionsorganismus bereitzustellen.

Demgemäß wurden in dem Ascomyceten Ashbya gossypii sechs Gene (rib-Gene), die für Enzyme der Riboflavin-Biosynthese ausgehend von GTP codieren, gefunden und isoliert.

Die Erfindung betrifft die folgenden DNA-Sequenzen:

DNA-Sequenzen, die für ein Polypeptid mit der in SEQ ID NO: 2 dargestellten Aminosäuresequenz codieren oder für ein Analoges oder Derivat des Polypeptids gemäß SEQ ID NO: 2, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA Sequenzen für das Analoge oder Derivat des Polypeptids zu mindestens 80 % homolog zu SEQ ID NO: 1 sind..

DNA-Sequenzen, die für ein Polypeptid mit der in SEQ ID NO: 4 dargestellten Aminosäuresequenz codieren oder für ein Analoges oder Derivat des Polypeptids gemäß SEQ ID NO: 4, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA Sequenzen für das Analoge oder Derivat des Polypeptids zu mindestens 80 % homolog zu SEQ ID NO: 3 sind.

DNA-Sequenzen, die für ein Polypeptid mit der in SEQ ID NO: 6 dargestellten Aminosäuresequenz codieren oder für ein Analoges oder Derivat des Polypeptids gemäß SEQ ID NO: 6, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA Sequenzen für das Analoge oder Derivat des Polypeptids zu mindestens 80 % homolog zu SEQ ID NO: 5 sind.

DNA-Sequenzen, die für ein Polypeptid mit der in SEQ ID NO: 8 dargestellten Aminosäuresequenz codieren oder für ein Analoges oder Derivat des Polypeptids gemäß SEQ ID NO: 8, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA Sequenzen für das Analoge oder Derivat des Polypeptids zu mindestens 80 % homolog zu SEQ ID NO: 7 sind.

DNA-Sequenzen, die für ein Polypeptid mit der in SEQ ID NO: 10 dargestellten Aminosäuresequenz codieren oder für ein Analoges oder Derivat des Polypeptids gemäß SEQ ID NO: 10, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA Sequenzen für das Analoge oder Derivat des Polypeptids zu mindestens 80 % homolog zu SEQ ID NO: 9 sind.

DNA-Sequenzen, die für ein Polypeptid mit der in SEQ ID NO: 12 dargestellten Aminosäuresequenz codieren oder für ein Analoges oder Derivat des Polypeptids gemäß SEQ ID NO: 12, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA Sequenzen für das Analoge oder Derivat des Polypeptids zu mindestens 80 % homolog zu SEQ ID NO: 11 sind.

Die Gene und ihre Genprodukte (Polypeptide) sind im Sequenzprotokoll mit ihrer Primärstruktur aufgeführt und haben folgende Zuordnung:
SEQ ID NO: 1 : rib 1-Gen
SEQ ID NO: 2 : rib 1-Genprodukt (GTP-cyclohydrolase II)
SEQ ID NO: 3 : rib 2-Gen
SEQ ID NO: 4 : rib 2-Genprodukt (DRAP-Deaminase)
SEQ ID NO: 5 : rib 3-Gen
SEQ ID NO: 6 : rib 3-Genprodukt (DBP-Synthase)
SEQ ID NO: 7 : rib 4-Gen
SEQ ID NO: 8 : rib 4-Genprodukt (DMRL-Synthase)
SEQ ID NO: 9 : rib 5-Gen
SEQ ID NO: 10: rib 5-Genprodukt (Riboflavin-Synthase)
SEQ ID NO: 11: rib 7-Gen
SEQ ID NO: 12: rib 7-Genprodukt (HTP-Reductase)

Guanosintriphosphat (GTP) wird durch GTP-Cyclohydrolase II (rib 1-Genprodukt) zu 2,5-Diamino-6-ribosylamino-4-(3H)-pyrimidinon-5-phosphat umgewandelt. Diese Verbindung wird anschließend durch rib 7-Genprodukt zu 2,5-Diamino-ribitylamino-2,4 (1H,3H)-pyrimidin-5-phosphat reduziert und dann durch rib 2-Genprodukt zum 5-Amino-6-ribitylamino-2,4 (1H,3H)-pyrimidindion deaminiert. Anschließend wird in einer rib 4-Genprodukt katalysierten Reaktion die C4-Verbindung DBP hinzugefügt und es entsteht 6,7-Dimethyl-8-ribityllumazin (DMRL), aus dem in der rib 5-Genprodukt katalysierten Reaktion Riboflavin entsteht. Die C4-Verbindung DBP (L-3,4-Dihydroxy-2-butanon-4-phosphat) wird aus D-Ribulose-5-phosphat in einer rib 3-Genprodukt katalysierten Reaktion gebildet.

Die in SEQ ID NO: 1,3,5,7,9,11 beschriebenen DNA-Sequenzen codieren für die Polypeptide, die in SEQ ID NO: 2,4,6,8,10,12 beschrieben sind.

Außer den im Sequenzprotokoll genannten DNA-Sequenzen sind auch solche geeignet, die infolge der Degeneration des genetischen Codes eine andere DNA Sequenz besitzen, jedoch für das gleiche Polypeptid codieren.

Weiterhin sind auch solche DNA Sequenzen Gegenstand der Erfindung, die für ein Genprodukt (Polypeptid) mit anderer als der im Sequenzprotokoll aufgeführten Primärstruktur codieren, solange das Genprodukt noch im wesentlichen die gleichen biologischen Eigenschaften wie das im Sequenzprotokoll genannte Genprodukt besitzt. Unter biologischen Eigenschaften sind vor allem die die Biosynthese von Riboflavin bewirkenden enzymatischen Aktivitäten zu verstehen.

Solche veränderten Genprodukte mit im wesentlichen gleichen biologischen Eigenschaften sind durch Deletion oder Hinzufügen von einer oder mehreren Aminosäuren oder Peptiden oder durch Austausch von Aminosäuren durch andere Aminosäuren erhältlich oder können aus anderen Organismen als Ashbya gossypii isoliert werden.

Die DNA-Sequenzen, die für die veränderten Genprodukte codieren, sind zu den DNA-Sequenzen gemäß Sequenzprotokoll in der Regel zu 80 oder mehr Prozent homolog. Solche DNA-Sequenzen lassen sich ausgehend von den in SEQ ID NO: 1, 3, 5, 7, 9, 11 beschriebenen DNA-Sequenzen, beispielsweise mit üblichen Hybridisierverfahren oder der PCR-Technik aus anderen Eukaryonten als Ashbya gossypii isolieren. Diese DNA-Sequenzen hybridisieren unter Standardbedingungen mit den in SEQ ID NO: 1, 3, 5, 7, 9, 11 beschriebenen DNA-Sequenzen.

Unter Standardbedingungen sind beispielsweise Temperaturen zwischen 42 und 58°C in einer wäßrigen Pufferlösung mit einer Konzentration zwischen 0,1 und 1 x SSC (1 x SSC: 0,15M NaCl, 15mM Natriumcitrat pH 7,2) zu verstehen. Die experimentellen Bedingungen für DNA-Hybridisierungen sind in Lehrbüchern der Gentechnik, beispielsweise in Sambrook et al., "Molecular Cloning", Cold Spring Harbor Laboratory, 1989, beschrieben.

Ein weiterer Gegenstand der Erfindung sind Regulationssequenzen, insbesondere Promotorsequenzen, die in 5'-Richtung vor den für die entsprechenden Polypeptid codierenden DNA-Sequenzen liegen. Die Regulationssequenzen sind im Sequenzprotokoll aufgeführt und im folgenden näher erläutert.

Regulationssequenz für rib 1-Gen:
SEQ ID NO: 1 Nukleotid 1-242

Regulationssequenz für rib 2-Gen:
SEQ ID NO: 3 Nukleotid 1-450

Regulationssequenz für rib 3-Gen:
SEQ ID NO: 5 Nukleotid 1-314

Regulationssequenz für rib 4-Gen:
SEQ ID NO: 7 Nukleotid 1-270

Regulationssequenz für rib 5-Gen:
SEQ ID NO: 9 Nukleotid 1-524

Regulationssequenz für rib 7-Gen:
SEQ ID NO: 11 Nukleotid 1-352

Die Regulationssequenzen können auch noch in 5'- und/oder 3'-Richtung verkürzt werden, ohne daß ihre Funktion wesentlich nachläßt.

Essentiell für die Regulationswirkung sind in der Regel Fragmente von 30 bis 100, bevorzugt 40 bis 70 Nukleotiden aus den oben angegebenen Sequenzbereichen.

Diese Regulationssequenzen können auch durch gerichtete Mutagenese im Vergleich zu den natürlichen Sequenzen in ihrer Funktion optimiert werden.

Die erfindungsgemäßen Regulationssequenzen eignen sich für die Überexpression von Genen in Ashbya, insbesondere von Genen, die für die Riboflavin-Biosynthese verantwortlich sind.

Weiterhin sind Gegenstand der Erfindung Expressionsvektoren, die eine oder mehrere der erfindungsgemäßen DNA-Sequenzen enthalten. Solche Expressionsvektoren erhält man, indem man die erfindungsgemäßen DNA-Sequenzen mit geeigneten funktionellen Regulationssignalen versieht. Solche Regulationssignale sind DNA-Sequenzen, die für die Expression verantwortlich sind, beispielsweise Promotoren, Operatoren, Enhancer, ribosomale Bindungsstellen, und die vom Wirtsorganismus erkannt und bedient werden.

Gegebenenfalls können noch weitere Regulationssignale, die beispielsweise Replikation oder Rekombination der rekombinanten DNA im Wirtsorganismus steuern, Bestandteil des Expressionsvektors sein.

Ebenso gehören die mit den erfindungsgemäßen DNA-Sequenzen oder Expressionsvektoren transformierten Wirtsorganismen zum Gegenstand der Erfindung. Bevorzugt werden als Wirtsorganismen eukaryontische Organismen, besonders bevorzugt solche der Gattung Saccharomyces, Candida, Pichia, Eremothecium oder Ashbya verwendet. Besonders bevorzugte Arten sind Saccharomyces cerevisiae, Candida flaveri, Candida famata, Eremothecium ashbyii und Ashbya gossypii.

Weiterhin gehört zur Erfindung ein rekombinantes Herstellverfahren für Riboflavin, in dem die erfindungsgemäßen transformierten Wirtsorganismen in an sich bekannter Weise durch Fermentation gezüchtet werden und das während der Fermentation gebildete Riboflavin aus dem Fermentationsmedium isoliert und gegebenenfalls gereinigt wird.

Die rib-Gene und -Genprodukte lassen sich wie im Beispiel und im Sequenzprotokoll beschrieben isolieren und charakterisieren.

### Beispiel 1

### Isolierung der Ashbya gossypii Riboflavin Biosynthese Gene (rib-Gene)

### a. Konstruktion einer Ashbya gossypii cDNA-Bank

Gesamt RNA wurde aus dem Mycel des Riboflavin überproduzierenden Stammes Ashbya gossypii ATCC 10195 nach Züchtung auf YEPD Medium (Sherman et al., "Methods in yeast genetics", Cold Spring Harbor, New York, 1989) in der späten logarithmischen Wachstumsphase extrahiert.

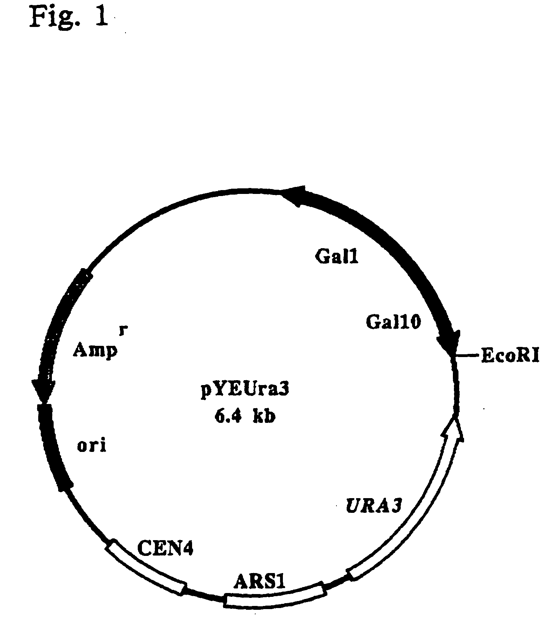

Poly(A)⁺ RNA wurde durch zweimalige Adsorption und Elution an oligo(dT)-Cellulose gereinigt (Aviv und Leder, Proc. Natl. Acad. Sci. USA 69,1972, 1408-1412). Die cDNA wurde nach der allgemeinen Vorschrift von Gubler und Hoffmann isoliert ( Gene 25, 1983, 263) und synthetische EcoRI-Adaptoren wurden an die Enden der bluntend cDNA-Moleküle hinzugefügt. Die EcoRI nachgeschnittenen cDNA Fragmente wurden anschließend mittels T4 Polynukleotidkinase phosphoryliert und in den dephosphorylierten EcoRI geschnittenen Vektor pYEura3 kloniert (Fig. 1). pYEura3 (Clonetech Laboratories, Inc., Kalifornien) ist ein Hefe-Expressionsvektor, der die Galaktose-induzierbaren GAL1 und GAL10 Promotoren und URA, CEN4 und ARS1 beinhaltet. Diese Hefeelemente erlauben die Transformation und Expression klonierter DNA-Fragmente in Hefezellen.

Aliquots der Ligationsreaktion wurden benutzt um hochkompetente (Hanahan, DNA Cloning, ed. D.M. Glover; IRL Press, Oxford 1985, 109) E. coli XL1-Blue (Bullock et al., Biotechniques 5 (1987) 376-378) zu transformieren und Transformanden wurden auf Basis ihrer Ampicillinresistenz selektioniert.

Etwa 3 x 10⁵ ampicillinresistente Zellen wurden vereinigt, amplifiziert und daraus Plasmid-DNA isoliert (Birnboim und Doly, Nucleic Acids Res. 7, 1979, 1513).

b. Isolierung von Ashbya gossypii cDNA-Klonen, die für riboflavinbildende Enzyme codieren

cDNA-Klone von Ashbya gossypii, die für riboflavinbildende Enzyme codieren, wurden durch funktionelle Komplementation von Saccharomyces cerevisiae Mutanten, die in der Riboflavin-Biosynthese betroffen sind, isoliert.

Die Stämme AJ88 ( Mata leu2 his3 rib1::URA3 ura3-52), AJ115 (Matalpha leu2 inos1 rib2::URA3 ura3-52), AJ71 (Matalpha leu2 inosl rib3::URA3 ura3-52), AJ106 (Matalpha leu2 inos1 rib4::URA3 ura3-52), AJ66 (Mata canR inos1 rib5::URA3 ura3-52) und AJ121 (Matalpha leu2 inos1 rib7::URA3 ura3-52) sind mutierte Stämme, die durch Zerstörung eines der sechs Gene (RIB1 bis RIB5 und RIB7), die in die Riboflavinbiosynthese bei Saccharomyces cerevisiae involviert sind.

Diese Stämme wurden jeweils mit 25 µg cDNA aus der Ashbya gossypii cDNA-Bank transformiert und auf festem Galaktose-haltigem Medium ohne Riboflavin ausplattiert. Nach ungefähr einer Woche Wachstum wurden Rib+ Transformanden von den Kulturschalen isoliert.

Jeweils eine Transformande von jeder transformierten Mutante (Rib1+, Rib2+, Rib3+, Rib4+, Rib5+ und Rib7+) wurde analysiert und in allen Fällen wurde gefunden, daß der Rib+ Phänotyp nur in Galaktosemedium, nicht jedoch in Glucosemedium exprimiert war.

Diese Ergebnisse belegen, daß der Rib+ Phänotyp unter der Kontrolle des plasmidständigen galaktoseinduzierbaren GAL10 Promotors exprimiert wurde.

Plasmid-DNA wurde aus den Rib1+, Rib2+, Rib3+, Rib4+, Rib5+ und Rib7+ Transformanden durch Transformation von E. coli isoliert und pJR715, pJR669, pJR788, pJR733, pJR681 und pJR827 genannt.

Partialsequenzierung der in diesen Plasmiden enthaltenen cDNA-Insertionen bestätigte, daß sie für Proteine codieren, die analog zu Proteinen der Rib-Genprodukte aus Saccharomyces sind.

c. Isolierung von Ashbya gossypii genomischen Klonen, die für riboflavinbildende Enzyme codieren

Um die genomischen Kopien der riboflavinbildenden Gene von Ashbya gossypii zu isolieren wurde eine genomische Bank von Ashbya gossypii ATCC 10195 in dem Cosmid superCosl (Stratagene Cloning Systems, Kalifornien) angelegt und mit ³²P-markierten Proben, die von den cDNA Kopien der RIB1, RIB2, RIB3, RIB4, RIB5 und RIB7 Gene von Ashbya gossypii abgeleitet waren, gescreent.

Cosmid Klone mit RIB1, RIB2, RIB3, RIB4, RIB5 und RIB7 DNA wurden isoliert durch Koloniehybridisierung (Grunstein und Hogness, Proc. Natl. Acad. Sci. USA 72, 1975, 3961-3965). weitere Southern Analysen von enzymatisch gespaltener Cosmid DNA unter Verwendung der gleichen RIB-spezifischen cDNA Proben erlaubte die Identifizierung definierter Restriktionsfragmente, die die RIB1, RIB2, RIB3, RIB4, RIB5 und RIB7 Gene von Ashbya gossypii enthielten.

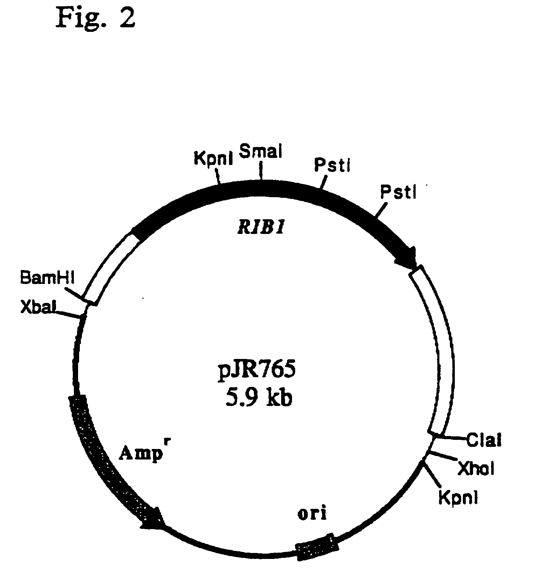

Ein 3,1 kb langes BamHI-ClaI DNA Fragment wurde gefunden, das das gesamte RIB1 Gen von Ashbya gossypii, codierend für GTP-Cyclohydrolase II enthielt. Dieses Fragment wurde aus einem Agarose Gel isoliert und in den BamHI und ClaI geschnittenen pBluescript KS (+) phagemid (Stratagene Cloning Systems) kloniert und lieferte so das Plasmid pJR765 (Fig.2).

Eine 1329 bp lange DNA Sequenz wurde erhalten (SEQ ID NO:1), die den RIB1 offenen Leserahmen von 906 bp, 242 bp von der 5'-nichtkodierenden Region und 181 bp von der 3'-nichtkodierenden Region enthielt.

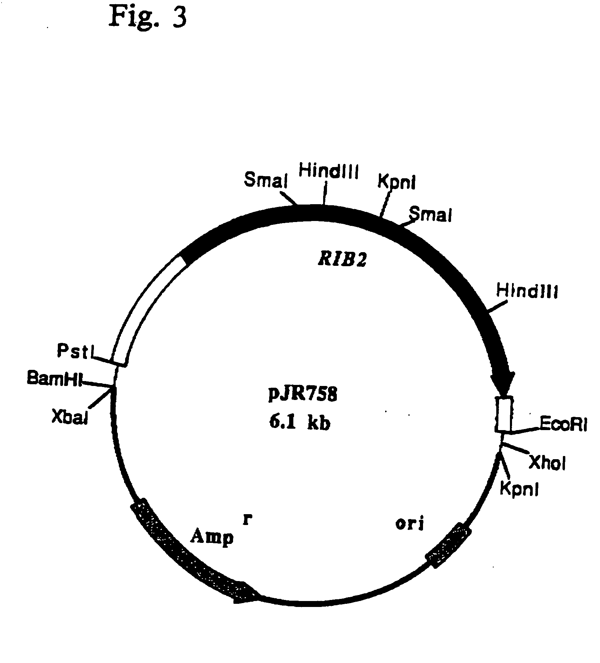

Das gesamte Ashbya gossypii RIB2 Gen, das für die DRAP-Deaminase codiert, wurde auf einem 3,0 kb langen EcoRI-PstI Fragment gefunden, das kloniert in pBluescript KS (+) das Plasmid PJR758 ergab (Fig.3).

Eine 2627 bp lange Region der EcoRI-PstI-Insertion mit dem offenen Leserahmen von RIB2 von 1830 bp, 450 bp der 5'-untranslatierten Region und 347 bp der 3'-untranslatierten Region wurde sequenziert (SEQ ID NO:3).

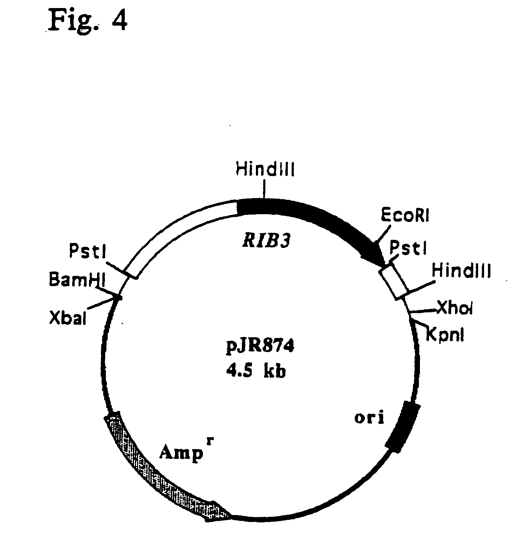

Das gesamte Ashbya gossypii RIB3 Gen, das für die DBP-Synthase codiert, wurde auf einem 1,5 kb langen PstI-HindIII Fragment gefunden, das kloniert in pBluescript KS (+) das Plasmid PJR790 ergab (Fig.4).

Eine 1082 bp lange Region der PstI-HindIII-Insertion mit dem offenen Leserahmen von RIB3 von 639 bp, 314 bp der 5'-untranslatierten Region und 129 bp der 3'-untranslatierten Region wurde sequenziert (SEQ ID NO:5).

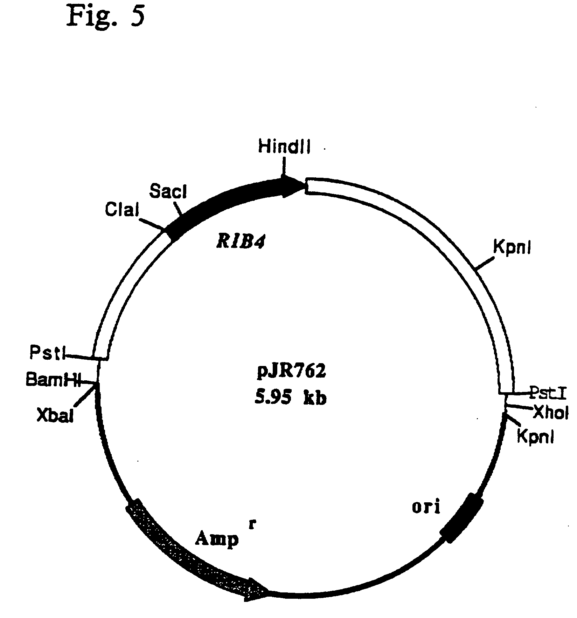

Das Ashbya gossypii RIB4 Gen, das für die DMRL-Synthase codiert, wurde auf einem 3,2 kb langen PstI-PstI Fragment gefunden, das kloniert in pBluescript KS (-) das Plasmid PJR762 ergab (Fig.5).

Eine 996 bp lange Region der PstI-PstI-Insertion mit dem offenen Leserahmen von RIB4 von 519 bp, 270 bp der 5'-untranslatierten) Region und 207 bp der 3'-untranslatierten Region wurde sequenziert (SEQ ID NO: 7).

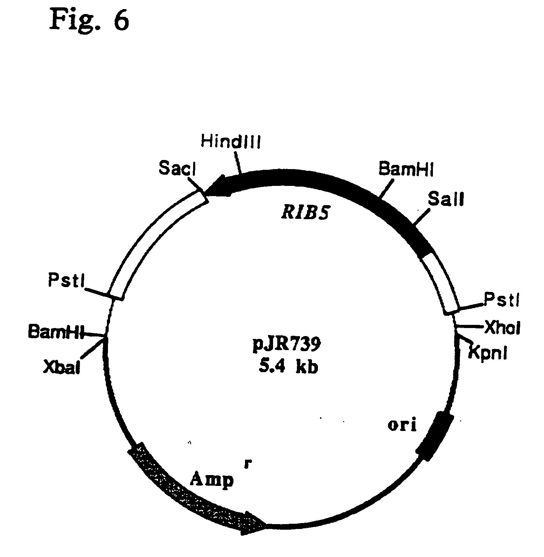

Das gesamte Ashbya gossypii RIB5 Gen, das für die Riboflavin-Synthase codiert, wurde auf einem 2,5 kb langen PstI-PstI Fragment gefunden, das kloniert in pBluescript KS (+) das Plasmid PJR739 (Fig.6) ergab.

Eine 1511 bp lange Region der PstI-PstI-Insertion mit dem offenen Leserahmen von RIB5 von 708 bp, 524 bp der 5'-untranslatierten Region und 279 bp der 3'-untranslatierten Region wurde sequenziert (SEQ ID NO:9).

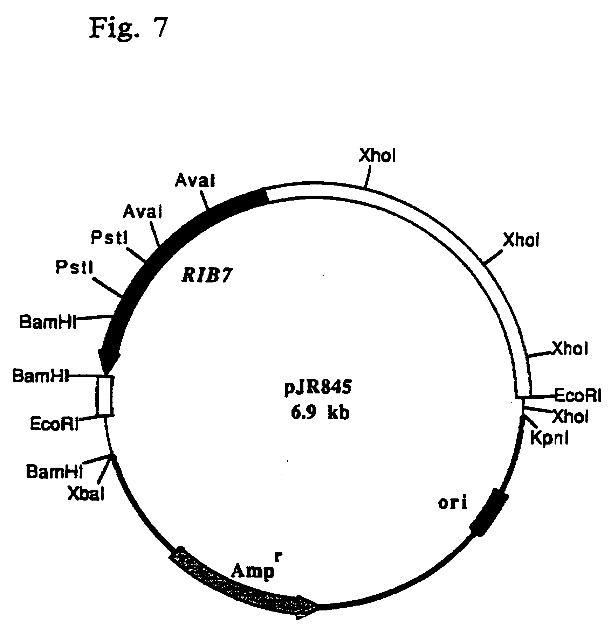

Schließlich wurde das Ashbya gossypii RIB7 Gen, das für die HTP-Reduktase codiert, auf einem 4,1 kb langen EcoRI-EcoRI-Fragment gefunden, das kloniert in pBluescript KS (+) das Plasmid PJR845 ergab (Fig.7).

Eine 1596 bp lange Region der EcoRI-EcoRI-Insertion mit dem offenen Leserahmen von RIB7 von 741 bp, 352 bp der 5'-untranslatierten Region und 503 bp der 3'-untranslatierten Region wurde sequenziert (SEQ ID NO:11).

### Beispiel 2

### mRNA Analyse der Ashbya gossypii RIB-Gene

Um die RIB spezifischen Transkripte zu identifizieren wurden Northern Analysen durchgeführt. Gesamt RNA wurde aus dem Ashbya gossypii Stamm ATCC 10195 wie in Beispiel 1 beschrieben, isoliert. Die RNA Proben des Stammes (5 µg) wurden elektrophoretisch aufgetrennt auf 0,8% Agarose-Formaldehyd-Gelen zusammen mit RNA-Größenmarkern und unter Vakuum auf Nylonmembrane geblottet (Thomas, Proc. Natl. Acad. Sci. USA, 77, 1980, 5201-5205).

Die Nylonmembranen wurden unabhängig voneinander mit ³²P-markierten RIB-spezifischen DNA-Proben bei 42°C in 5xSSC und in Gegenwart von 50 % Formamid hybridisiert. Das Ashbya gossypii RIB1 Gen wird als unique Message von etwa 1150 Nukleotiden exprimiert, was in beiden Stämmen durch eine 0,7 kbp lange SmaI-SacI Probe aus dem Plasmid pJR765 (Fig. 8) nachgewiesen wurde.

Analog wurden unique 1900 Nukleotide lange RIB2- , 900 Nukleotide lange RIB3-, 800 Nukleotide lange RIB4-, 1050 Nukleotide lange RIB5- und 1000 Nukleotide lange RIB7-Transkripte in den Blots mit Hilfe eines 0,5 kbp langen SmaI-SmaI-Fragments aus pJR758, eines 0,6 kbp langen HindIII-KpnI-Fragments aus pJR790, eines 0,5 kbp langen ScaI-HindIII Fragments aus pJR739 und eines 0,3 kbp langen PstI-PstI-Fragments aus pJR845 als spezifischer Probe nachgewiesen.

### Beispiel 3

### Expression der Ashbya gossypii RIB-Gene in Saccharomyces cerevisiae

Wie in Beispiel 1 beschrieben, können gut untersuchte Mutanten von Saccharomyces cerevisiae, die in einer Stufe der Riboflavinbiosynthese defekt sind, auf Kulturmedien ohne Riboflavin wachsen, wenn sie ein Plasmid tragen, das für die komplementierenden Enzyme von Ashbya codiert. Um die Funktion der Ashbya gossypii RIB Genprodukte zu testen wurden flavinbildende Enzymaktivitäten in zellfreien Extrakten von S. cerevisiae- Mutanten gemessen, die eines der Expressionsplasmide pJR715, pJR669, pJR788, pJR733, pJR681 und pJR827 trugen.

Diese in Beispiel 1 beschriebenen von pYEura3 abgeleiteten Plasmide enthalten Ashbya gossypii RIB-spezifische cDNA-Fragmente unter der Kontrolle des galaktoseinduzierbaren GAL10 Promotors.

Zellfreie Proteinextrakte von S. cerevisiae wurden aus Kulturen gewonnen, die in Flüssigmedium bis zu einer optischen Dichte von etwa 2 OD gewachsen waren.

Die Zellen wurden geerntet, mit kaltem 20 mM Tris HCl, pH 7,5 gewaschen und im gleichen Puffer, der mit 1 mM Phenylethylsulfonylfluorid supplementiert war, resuspendiert.

Zell-Lysate wurden durch Vortexen in Gegenwart von Glaskugeln und Zentrifugation bei 3000 g für 20 min. bei 4°C hergestellt.

GTP-Cyclohydrolase II, DRAP-Deaminase, DBP-Synthase, DMRL-Synthase, Riboflavin-Synthase und HTP-Reduktase Enzymaktivitäten wurden bestimmt wie in der Literatur beschrieben (Shavlovsky et al, Arch. Microbiol. 124 1980, 255-259; Richter et al., J. Baceriol. 175, 1993, 4045-4051; Klein und Bacher, Z. Naturforsch. 35b, 1980, 482-484; Richter et al. J. Bacteriol. 174, 1992, 4050-4056; Nielsen et al. J. Biol. Chem. 261, 1986, 3661; Plaut und Harvey, Methods Enzymol. 18B, 1971, 515-538; Hollander und Brown, Biochem. Biophys. Res. Commun. 89, 1979, 759-763; Shavlovski et al., Biochim. Biophys. Acta, 428, 1976, 611-618).

Protein-wurde nach der Methode von Peterson quantifiziert (Anal. Biochem. 83, 1977, 346-356). Wie aus Tab. 1 ersichtlich, bewirkt . das Plasmid pJR715 die Expression von GTP-Cyclohydrolase II Aktivität in der S. cerevisiae Mutante AJ88. Weiterhin ist diese Aktivität nur vorhanden in Zellen, die auf Galaktosemedium gewachsen sind, was darauf hinweist, daß die RIB1 cDNA Expression von Ashbya gossypii unter der Kontrolle des galaktoseinduzierbaren GAL10-Promotors erfolgt.

Daher belegen diese Ergebnisse, daß RIB1 für die GTP-Cyclohydrolase II in Ashbya gossypii codiert. Auf analoge Art wurde gezeigt daß RIB2 für DRAP-Deaminase, RIB3 für DBP-Synthase, RIB4 für DMRL-Synthase, RIB5 für Riboflavinsynthase und RIB7 für HTP-Reduktase in diesem Pilz codiert.

**Tab. 1**

| GTP-Cyclohydrolase II Aktivität der S. cerevisiae RIB1 Mutante AJ88 und ihrer Transformanden. | | | |
|---|---|---|---|
| Stamm | Plasmid | GTP-Cyclohydrolase II U/mg Protein**) | |
| | | Glucose | Galaktose |
| X 2180-1A* | - | 0,48 | 0,34 |
| AJ 88 | - | n.d. | n.d. |
| AJ 88 | pIR715 | n.d. | 21,60 |

| | | | |
|---|---|---|---|
| n.d.:not detected | | | |
| *) Wildtyp | | | |
| **) Einheiten GTP-Cyclohydrolase II Aktivitäten 1U katalysiert die Bildung von 1 nmol HTP pro Stunde | | | |

**Tab. 2**

| DRAP-Deaminase Aktivität der S. cerevisiae RIB2 Mutante AJ115 und ihrer Transformanden. | | | |
|---|---|---|---|
| Stamm | Plasmid | DRAP-Deaminase U/mg Protein *) | |
| | | Glucose | Galaktose |
| X 2180-1A | - | 0,45 | 0,38 |
| AJ 115 | - | n.d. | n.d. |
| AJ 115 | pIR669 | n.d. | 53,22 |

| | | | |
|---|---|---|---|
| n.d.:not detected | | | |
| *) 1U katalysiert die Bildung von 1 nmol ARAP pro Stunde | | | |

**Tab. 3**

| DBP-Synthase Aktivität der S. cerevisiae RIB3 Mutante AJ71 und ihrer Transformanden. | | | |
|---|---|---|---|
| Stamm | Plasmid | DBP-Synthase U/mg Protein*) | |
| | | Glucose | Galaktose |
| X 2180-1A | - | 0,80 | 0,75 |
| AJ 71 | - | n.d. | n.d. |
| AJ 71 | pIR788 | n.d. | 25,19 |

| | | | |
|---|---|---|---|
| n.d.:not detected | | | |
| *) 1U katalysiert die Bildung von 1 nmol DBP pro Stunde | | | |

**Tab. 4**

| GTP-Cyclohydrolase II Aktivität der S. cerevisiae RIB4 Mutante AJ106 und ihrer Transformande. | | | |
|---|---|---|---|
| Stamm | Plasmid | DMRL-Synthase U/mg Protein *) | |
| | | Glucose | Galaktose |
| X 2180-1A | - | 2,04 | 1,73 |
| AJ 106 | - | n.d. | n.d. |
| AJ 106 | pIR733 | n.d. | 86,54 |

| | | | |
|---|---|---|---|
| n.d.:not detected | | | |
| *) 1U katalysiert die Bildung von 1 nmol DMRL pro Stunde | | | |

**Tab. 5**

| Riboflavin-Synthase Aktivität der S. cerevisiae RIB5 Mutante AJ66 und ihrer Transformande. | | | |
|---|---|---|---|
| Stamm | Plasmid | Riboflavin-Synthase U/mg Protein *) | |
| | | Glucose | Galaktose |
| X 2180-1A | - | 4,41 | 3,80 |
| AJ 66 | - | n.d. | n.d. |
| AJ 66 | pIR681 | n.d. | 164,20 |

| | | | |
|---|---|---|---|
| n.d.:not detected | | | |
| *) 1U katalysiert die Bildung von 1 nmol Riboflavin pro Stunde | | | |

**Tab. 6**

| HTP-Reduktase Aktivität der S. cerevisiae RIB7 Mutante AJ121 und ihrer Transformande. | | | |
|---|---|---|---|
| Stamm | Plasmid | HTP-Reductase U/mg Protein *) | |
| | | Glucose | Galaktose |
| X 2180-1A | - | 1,86 | 2,54 |
| AJ 121 | - | n.d. | n.d. |
| AJ 121 | pIR827 | n.d. | 46,21 |

| | | | |
|---|---|---|---|
| n.d.:not detected | | | |
| *) 1U katalysiert die Bildung von 1 nmol DRAP pro Stunde | | | |

## Patentansprüche

1. DNA-Sequenzen, die für ein Polypeptid mit der in SEQ ID NO: 2 dargestellten Aminosäuresequenz (GTP-Cyclohydrolase II) codieren oder für ein Analoges oder Derivat des Polypeptids gemäß SEQ ID NO: 2, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA-Sequenzen für das Analoge oder Derivat des Polypeptids zu mindestens 80 % homolog zu SEQ ID NO: 1 sind.

2. DNA-Sequenzen, die für ein Polypeptid mit der in SEQ ID NO: 4 dargestellten Aminosäuresequenz (DRAP-Deaminase) codieren oder für ein Analoges oder Derivat des Polypeptids gemäß SEQ ID NO: 4, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA-Sequenzen für das Analoge oder Derivat des Polypeptids zu mindestens 80 % homolog zu SEQ ID NO: 3 sind.

3. DNA-Sequenzen, die für ein Polypeptid mit der in SEQ ID NO: 6 dargestellten Aminosäuresequenz (DBP-Synthase) codieren oder für ein Analoges oder Derivat des Polypeptids gemäß SEQ ID NO: 6, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA-Sequenzen für das Analoge oder Derivat des Polypeptids zu mindestens 80 % homolog zu SEQ ID NO: 5 sind.

4. DNA-Sequenzen, die für ein Polypeptid mit der in SEQ ID NO: 8 dargestellten Aminosäuresequenz (DMRL-Synthase) codieren oder für ein Analoges oder Derivat des Polypeptids gemäß SEQ ID NO: 8, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA-Sequenzen für das Analoge oder Derivat des Polypeptids zu mindestens 80 % homolog zu SEQ ID NO: 7 sind.

5. DNA-Sequenzen, die für ein Polypeptid mit der in SEQ ID NO: 10 dargestellten Aminosäuresequenz (Riboflavin-Synthase) codieren oder für ein Analoges oder Derivat des Polypeptids gemäß SEQ ID NO: 10, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA-Sequenzen für das Analoge oder Derivat des Polypeptids zu mindestens 80 % homolog zu SEQ ID NO: 9 sind.

6. DNA-Sequenzen, die für ein Polypeptid mit der in SEQ ID NO: 12 dargestellten Aminosäuresequenz (HTP-Reductase) codieren oder für ein Analoges oder Derivat des Polypeptids gemäß SEQ ID NO: 12, worin eine oder mehrere Aminosäuren deletiert, hinzugefügt oder durch andere Aminosäuren substituiert worden sind, ohne die enzymatische Wirkung des Polypeptids wesentlich zu reduzieren, mit der Maßgabe, daß die DNA-Sequenzen für das Analoge oder Derivat des Polypeptids zu mindestens 80 % homolog zu SEQ ID NO: 11 sind.

7. Expressionsvektor, enthaltend eine oder mehrere DNA-Sequenzen gemäß Anspruch 1 bis 6.

8. Nicht-menschlicher Wirtsorganismus der mit einem Expressionssystem gemäß Anspruch 7 transformiert worden ist.

9. Rekombinantes Herstellverfahren für Riboflavin, **dadurch gekennzeichnet, daß** ein Wirtsorganismus gemäß Anspruch 8 verwendet wird.

## Claims

1. A DNA sequence which codes for a polypeptide with the amino-acid sequence (GTP cyclohydrolase II) depicted in SEQ ID NO: 2, or for an analog or derivative of the polypeptide shown in SEQ ID NO: 2, in which one or more amino acids have been deleted, added or replaced by other amino acids, without essentially reducing the enzymatic action of the polypeptide with the proviso that the DNA sequence for the analog or derivative of the polypeptide is homologous to the extent of at least 80% with SEQ ID NO: 1.

2. A DNA sequence which codes for a polypeptide with the amino-acid sequence (DRAP deaminase) depicted in SEQ ID NO: 4, or for an analog or derivative of the polypeptide shown in SEQ ID NO: 4, in which one or more amino acids have been deleted, added or replaced by other amino acids, without essentially reducing the enzymatic action of the polypeptide with the proviso that the DNA sequence for the analog or derivative of the polypeptide is homologous to the extent of at least 80% with SEQ ID NO: 3.

3. A DNA sequence which codes for a polypeptide with the amino-acid sequence (DBP synthase) depicted in SEQ ID NO: 6, or for an analog or derivative of the polypeptide shown in SEQ ID NO: 6, in which one or more amino acids have been deleted, added or replaced by other amino acids, without essentially reducing the enzymatic action of the polypeptide with the proviso that the DNA sequence for the analog or derivative of the polypeptide is homologous to the extent of at least 80% with SEQ ID NO: 5.

4. A DNA sequence which codes for a polypeptide with the amino-acid sequence (DMRL synthase) depicted in SEQ ID NO: 8, or for an analog or derivative of the polypeptide shown in SEQ ID NO: 8, in which one or more amino acids have been deleted, added or replaced by other amino acids, without essentially reducing the enzymatic action of the polypeptide with the proviso that the DNA sequence for the analog or derivative of the polypeptide is homologous to the extent of at least 80% with SEQ ID NO: 7.

5. A DNA sequence which codes for a polypeptide with the amino-acid sequence (riboflavin synthase) depicted in SEQ ID NO: 10, or for an analog or derivative of the polypeptide shown in SEQ ID NO: 10, in which one or more amino acids have been deleted, added or replaced by other amino acids, without essentially reducing the enzymatic action of the polypeptide with the proviso that the DNA sequence for the analog or derivative of the polypeptide is homologous to the extent of at least 80% with SEQ ID NO: 9.

6. A DNA sequence which codes for a polypeptide with the amino-acid sequence (HTP reductase) depicted in SEQ ID NO: 12, or for an analog or derivative of the polypeptide shown in SEQ ID NO: 12, in which one or more amino acids have been deleted, added or replaced by other amino acids, without essentially reducing the enzymatic action of the polypeptide with the proviso that the DNA sequence for the analog or derivative of the polypeptide is homologous to the extent of at least 80% with SEQ ID NO: 11.

7. An expression vector containing one or more DNA sequences as claimed in claims 1 to 6.

8. A non-human host organism which has been transformed with an expression system as claimed in claim 7.

9. A recombinant process for preparing riboflavin, which makes use of a host organism as claimed in claim 8.

## Revendications

1. Séquences d'ADN, qui codent pour un polypeptide ayant la séquence d'acides aminés représentée dans SEQ ID NO: 2 (GTP-cyclohydrolase II) ou pour un analogue ou dérivé du polypeptide selon SEQ ID NO: 2, tandis qu'un ou plusieurs acides aminés ont été délétés, ajoutés ou substitués par d'autres acides aminés, sans réduire substantiellement l'activité enzymatique du polypeptide, avec pour condition que les séquences d'ADN sont homologues de SEQ ID NO: 1 pour au moins 80 % pour l'analogue ou le dérivé du polypeptide.

2. Séquences d'ADN, qui codent pour un polypeptide ayant la séquence d'acides aminés représentée dans SEQ ID NO: 4 (DRAP-déaminase) ou pour un analogue ou dérivé du polypeptide selon SEQ ID NO: 4, tandis qu'un ou plusieurs acides aminés ont été délétés, ajoutés ou substitués par d'autres acides aminés, sans réduire substantiellement l'activité enzymatique du polypeptide, avec pour condition que les séquences d'ADN sont homologues de SEQ ID NO: 3 pour au moins 80 % pour l'analogue ou le dérivé du polypeptide.'

3. Séquences d'ADN, qui codent pour un polypeptide ayant la séquence d'acides aminés représentée dans SEQ ID NO: 6 (DBP-synthase) ou pour un analogue ou dérivé du polypeptide selon SEQ ID NO: 6, tandis qu'un ou plusieurs acides aminés ont été délétés, ajoutés ou substitués par d'autres acides aminés, sans réduire substantiellement l'activité enzymatique du polypeptide, avec pour condition que les séquences d'ADN sont homologues de SEQ ID NO: 5 pour au moins 80 % pour l'analogue ou le dérivé du polypeptide.

4. Séquences d'ADN, qui codent pour un polypeptide ayant la séquence d'acides aminés représentée dans SEQ ID NO: 8 (DMRL-synthase) ou pour un analogue ou dérivé du polypeptide selon SEQ ID NO: 8, tandis qu'un ou plusieurs acides aminés ont été délétés, ajoutés ou substitués par d'autres acides aminés, sans réduire substantiellement l'activité enzymatique du polypeptide, avec pour condition que les séquences d'ADN sont homologues de SEQ ID NO: 7 pour au moins 80 % pour l'analogue ou le dérivé du polypeptide.

5. Séquences d'ADN, qui codent pour un polypeptide ayant la séquence d'acides aminés représentée dans SEQ ID NO: 10 (riboflavine-synthase) ou pour un analogue ou dérivé du polypeptide selon SEQ ID NO: 10, tandis qu'un ou plusieurs acides aminés ont été délétés, ajoutés ou substitués par d'autres acides aminés, sans réduire substantiellement l'activité enzymatique du polypeptide, avec pour condition que les séquences d'ADN sont homologues de SEQ ID NO: 9 pour au moins 80 % pour l'analogue ou le dérivé du polypeptide.

6. Séquences d'ADN, qui codent pour un polypeptide ayant la séquence d'acides aminés représentée dans SEQ ID NO: 12 (HTP-réductase) ou pour un analogue ou dérivé du polypeptide selon SEQ ID NO: 12, tandis qu'un ou plusieurs acides aminés ont été délétés, ajoutés ou substitués par d'autres acides aminés, sans réduire substantiellement l'activité enzymatique du polypeptide, avec pour condition que les séquences d'ADN sont homologues de SEQ ID NO: 11 pour au moins 80 % pour l'analogue ou le dérivé du polypeptide.

7. Vecteur d'expression, contenant une ou plusieurs séquences d'ADN selon la revendication 1 à 6.

8. Organisme hôte non-humain qui a été transformé avec un système d'expression selon la revendication 7.

9. Procédé pour la préparation de riboflavine par recombinaison, **caractérisé par le fait qu'**on utilise un organisme hôte selon la revendication 8.
